# EUROPEAN PATENT APPLICATION

(11) **EP 3 459 529 A1**
(43) Date of publication of application: **27.03.2019**
(21) Application number: 17192261.0
(22) Date of filing: 20.09.2017
(51) Int. Cl.: A61K 9/16, C07K 16/24, A61K 39/00

(54) **PREPARATION OF SUSTAINED RELEASE SOLID DOSAGE FORMS COMPRISING ANTIBODIES BY SPRAY DRYING**

(71) Applicant: TILLOTTS PHARMA AG, 4310 Rheinfelden (CH)
(72) Inventor: BRUNO, Cristina, 4310 Rheinfelden (CH); VARUM, Felipe, 4310 Rheinfelden (CH); BRAVO, Roberto, 4310 Rheinfelden (CH)
(74) Representative: Kalhammer, Georg

(57) **Abstract**

The present invention relates to a method for preparing individual solid particles by spray drying, comprising antibodies and functional fragments thereof and sustained release polymers, suitable to be processed into sustained release solid dosage forms, optionally coated with a delayed release coating; the particles and solid dosage forms prepared by the method; and the use of the solid dosage forms in the topical treatment in the gastrointestinal tract of a patient.

## Description

The present invention relates to a method for preparing individual solid particles by spray drying, comprising antibodies and functional fragments thereof and sustained release polymers, suitable to be processed into sustained release solid dosage forms, optionally coated with a delayed release coating; the particles and solid dosage forms prepared by the method; and the use of the solid dosage forms in the topical treatment in the gastrointestinal tract of a patient.

### BACKGROUND

Biologically active polypeptides, in particular large polypeptides with antigen binding activity such as antibodies and functional fragments thereof, due to their intrinsic nature, are sensitive to any change in their environment, giving rise to inherent instability. Therefore, ensuring their stability and activity as well as the therapeutically efficacious release upon incorporation into a pharmaceutical composition is very challenging and yet paramount due to the prohibitive costs of such antibodies in quantities that allow their therapeutic application to a patient. Generally, this inherent instability of antibodies is independent of whether they are used to prepare a pharmaceutical composition in a liquid, gelatinous, semi-solid, solid or any other form. However, in particular for solid dosage forms, many processing steps in the preparation can be detrimental to stability and activity of an antibody or functional fragment thereof.

The use of solid dosage forms is very common for pharmaceutical compositions intended for enteral administration. Enteral administration, and especially oral administration, of solid dosage forms comprising biologically active polypeptides, due to convenience and safety, has become increasingly important in recent years, as it further allows, besides a systemic treatment, for the topical treatment of symptoms of diseases of the gastrointestinal tract (gastrointestinal diseases), as for example inflammatory bowel disease (IBD), colorectal cancer, diarrhea or microbial infections.

Many factors may affect the chemical and physical stability and thereby the activity of large biologically active polypeptides like antibodies and functional fragments thereof during the incorporation into a solid dosage form. Chemical instability of large polypeptides, e.g. in the form of fragmentation, oxidation, deamination, isomerization, disulfide bond formation or formation of acidic/basic species, is directly affected by excipients used in the solid dosage form, as well as by pH, physical stress and temperature during the preparation and later storage of the solid dosage form. Physical instability, e.g. in the form of denaturation, aggregation or adsorption, can result from shear stress, changes in temperature, or high pressure during preparation and later storage. Biological instability, e.g. in the form of proteolytic digestion or post-translational modification, can result from the exposure to proteases and other enzymes, as well as other biological factors able to affect the integrity of large polypeptides. The processing of antibodies and functional fragments thereof, in order to incorporate them into solid dosage forms, therefore poses major challenges, in particular with regard to the choice of individual excipients as well as with regard to the processing parameters. In addition to directly affecting stability and activity of the antibodies and functional fragments thereof, the choice of method for preparing the solid dosage form will also affect the properties of the resulting solid dosage form, i.e. its stability, integrity, quality and dissolution behavior.

Spray drying is a very widely applied technique used to dry aqueous or organic solutions, suspensions and emulsions for example in the food, chemical, pharmaceutical and biopharmaceutical industry. Spray drying involves the spraying of a liquid feed medium, in the form of a solution, suspension or emulsion into a hot stream of drying gas. The droplets formed by the atomisation process are dried through solvent evaporation to form particles, which are collected as dry particles. Spray drying uses a one-step process for formation and drying of particles. In this way the number of processing steps in the preparation of a solid pharmaceutical composition can be reduced, thus improving production efficiency and reducing costs, especially since spray drying is a continuous process, which can be easily automated. The characteristics of the spray-dried particles, in particular particle size, particle size distribution, particle shape and residual moisture content,can be easily controlled by the spray drier setup and process conditions. Spray drying thus is an excellent method for the production of dry powder formulations with fine or coarse particles as well as larger granules.

Spray drying can be used to increase the solubility of poorly soluble active agents. For example WO 03/043603 discloses pharmaceutical compositions comprising poorly soluble active agents, selected from small molecules, proteins, polypeptides and peptides, which are spray dried into amorphous and hollow particles comprising regions of poorly soluble agent embedded within the wall of said particles to improve dissolution rate of the particles. The particles may comprise 100 % drug or may comprise one or more excipients.

Antibodies can be spray dried from a solution into particles to form a powder for long-term storage (Bowen et al., Drying Technology, 31: 1441-1450, 2013). Compared to freeze drying, a widely used drying technique for antibodies, spray drying antibodies e.g. together with a carbohydrate sugar, can reduce energy consumption while ensuring comparable or greater stability of the antibodies compared to freeze drying.

In IBD, like ulcerative colitis or Crohn's disease, for the inflamed colonic mucosa to be exposed to an antibody concentration effective for topical treatment, the antibody must retain sufficient stability and activity until it is taken up by the target mucosa. In order to minimize antibody degradation in the colonic luminal fluids (Yadav et al., International Journal of Pharmaceutics, 2016. 502(1- 2): p. 181-187), a slow and controlled release of antibodies or functional fragments thereof from a solid dosage form is desirable. This would ensure the release of the antibody or functional fragment thereof from the solid dosage form at a rate that allows the effective uptake into mucosa and a continuous provision of antibody or functional fragment thereof over several hours and up to a day or even longer. Moreover, it would allow the treatment of a greater target area of inflamed mucosa, as it would allow the solid dosage form to release antibodies or functional fragments thereof while moving along the inside of the gastrointestinal tract. However, the release of antibodies or functional fragments from solid dosage forms may be further modulated by the use of polymers suitable for a prolonged release of the antibody or functional fragment thereof (i.e. sustained release polymeric binders).

Gastrointestinal transit, in particular, colonic transit of solid dosage forms shows a wide inter- and intra-individual variability (Varum et al., Int. J. Pharm., 2010. 395(1-2): p. 26-36). Furthermore, IBD such as ulcerative colitis or Crohn's disease can also influence transit time of dosage forms. It has been shown, that in some cases, colonic transit is prolonged in ulcerative colitis, in the areas proximal to the inflamed mucosa. Thus a dosage form will stay in those areas longer, before reaching the inflamed mucosa. If the antibody is not provided in a stable enough form this could mean premature antibody degradation leading to reduced levels of antibody reaching the distal colonic mucosa. On the other hand, transit through the inflamed area can be accelerated in some cases, which further complicates the design of a dosage form for an efficient antibody delivery to the ileum and large intestine.

Due to the biopharmaceutical advantages of multiparticulates over single units, such as longer colonic transit than single units and wider spread of dose in multiple small units (Varum et al., Int. J. Pharm., 2010. 395(1-2): p. 26-36), a multiparticulate drug delivery system would be a preferred choice. Single units of such a multiparticulate drug delivery system (multiparticulate solid dosage form) could be designed to achieve sustained antibody release.

Solid dosage forms can be prepared from spray-dried antibody particles by coating/encapsulating them with a polymer. Those particles could be used as such in the final drug product, or further processed in capsules, tablets, pellets and the like. For example WO 2013/075068 discloses a microparticle comprising a protein, e.g. an antibody, which is prepared by spray drying, and then coated with a biocompatible and biodegradable polymer. For that the spray-dried microparticles are suspended in a polymer solution, comprising a biocompatible and biodegradable polymer dissolved in an organic solvent, and again subjected to spray drying. WO 2009/090189 discloses a method for preparing powdered protein compositions, by spray drying a solution comprising more than about 50 mg/ml of a protein or peptide, e.g. an antibody, and at least one excipient like trehalose, sucrose and the like. In a further step, discrete spray dried particles or aggregates of particles of the powdered protein composition may then be coated with a coating. Suitable coatings can include polymers, such as PLGA to form a sustained release or delayed release pharmaceutical compositions. The above methods involving spray drying require multiple manufacturing steps for preparing a sustained release solid dosage form. The above methods thus are time and cost intensive and would expose an antibody or functional fragment thereof to excessive external stress, which can be detrimental its activity and stability.

There is a need for a method for preparing sustained release solid dosage forms by spray drying, comprising antibodies or functional fragments thereof, that minimizes loss of biological activity of the antibody or functional fragment thereof used for the preparation of the solid dosage forms. In particular, the method should minimize processing time and steps required for the preparation of the solid dosage forms, preserve stability and activity of the antibody or functional fragment thereof during the preparation, allow release over a prolonged period of time, and minimize interactions of the at least one antibody or functional fragment thereof with other excipients of the solid dosage forms that could limit antibody release and recovery.

### SUMMARY OF THE INVENTION

After testing a vast number of processing conditions and excipients the present inventors found an advantageous method for preparing particles comprising at least one antibody or functional fragment thereof, a buffer and at least one sustained release polymer by spray drying. The present inventors found such particles can be prepared in a one-step process, by spray drying an aqueous suspension comprising at least one sustained release polymer, a buffer and at least one antibody or functional fragment thereof, to form individual particles. Multiple of these particles can be combined into a multiparticulate solid dosage form. This method ensures a fast and straight forward preparation of these particles, preserves stability and activity of the antibodies or functional fragments thereof used for the preparation and allows for an optimal amount of the antibody or functional fragment thereof to be recovered from the particles in a sustained release manner. Moreover, a multiparticulate solid dosage form comprising a plurality of these particles can be prepared by a straight forward method. Finally, the method allows for the preparation of delayed release oral multiparticulate and/or single unit solid dosage forms comprising a delayed release coating to ensure drug release at the target site in the gastrointestinal tract of a patient.

Thus, the present invention provides a novel method for preparing particles, comprising at least one sustained release polymer, a buffer and as an active agent at least one antibody or functional fragment thereof, and optionally a plasticizer and/or a surfactant and/or filler, prepared by spray drying, as well as multiparticulate and single unit solid dosage forms prepared by combining a plurality of these particles. The present invention relates to the subject matter defined in the following items 1 to 111:
[1] A method for preparing particles comprising at least one antibody or functional fragment thereof as active agent, a buffer and at least one sustained release polymer, by spray drying; the method comprising the steps of
   a) preparing a feed liquid, comprising the at least one antibody or functional fragment thereof, the buffer and the at least one sustained release polymer, all in an aqueous suspension;
   b) spraying the feed liquid through a nozzle into a primary drying chamber to form droplets, wherein the droplets are dispersed in a drying gas;
   c) drying the droplets, by evaporating at least a portion of the liquid in the droplets into the drying gas, to form particles dispersed in the drying gas; and
   d) directing the drying gas with the particles dispersed therein, through a particle concentration means to recover the particles.
[2] Method according to item 1, wherein the sustained release polymer is not biodegradable.
[3] Method according to item 1 or 2, wherein the at least one sustained release polymer is selected from the group consisting of poly(ethyl acrylate, methyl methacrylate, trimethylammonioethyl methacrylate chloride) 1 : 2 : 0.1 (e.g. Eudragit® RS 30D); poly(ethyl acrylate, methyl methacrylate, trimethylammonioethyl methacrylate chloride) 1 : 2 : 0.2 (e.g. Eudragit® RL 30D); poly(ethylacrylate, methylmethacrylate) 2:1 (e.g. Eudragit® NE 30D, Eudragit® NE 40D, Eudragit® NM 30D) polyvinyl acetate; polyvinyl acetate aqueous dispersion stabilized with povidone and sodium lauryl sulfate (e.g. Kollicoat® SR 30D); aqueous dispersion consisting of about 27% polyvinyl acetate, 2.7% povidone and 0.3% sodium lauryl sulfate (i.e. Kollicoat® SR 30D); ethylcellulose aqueous dispersions (Aquacoat® ECD, Surelease®), hydroxypropyl methylcellulose (HPMC); HPMC 2208 type with a viscosity at 2 wt.-% in water at 20°C between 3 and 1,000 mPa·s, preferably HPMC 2208 type with a viscosity at 2 wt.-% in water at 20°C between 78 and 117 mPa·s (e.g. Methocel® K100); and combinations thereof.
[4] Method according to item 3, wherein the at least one sustained release polymer is poly(ethyl acrylate, methyl methacrylate, trimethylammonioethyl methacrylate chloride) 1 : 2 : 0.1 (e.g. Eudragit® RS 30D); HPMC 2208 type with a viscosity at 2 wt.-% in water at 20°C between 78 and 117 mPa·s (e.g. Methocel® K100); and combinations thereof; preferably provided as aqueous suspension (dispersion); more preferably poly(ethyl acrylate, methyl methacrylate, trimethylammonioethyl methacrylate chloride) 1 : 2 : 0.1.
[5] Method according to any of the preceding items, wherein the at least one sustained release polymer is dispersed in the feed liquid (suspension).
[6] Method according to any of the preceding items, wherein the at least one sustained release polymer is provided as an aqueous suspension (dispersion).
[7] Method according to any of the preceding items, wherein the functional antibody fragment is a Fab fragment, a F(ab')2 fragment, a Fab' fragment, an scFv, a dsFv, a VHH, a diabody, a triabody, a tetrabody, an Fc fusion protein or a minibody.
[8] Method according to any of the preceding items, wherein the at least one antibody or functional fragment thereof is selected from antibodies specific to tumor necrosis factor alpha (TNFα) and functional fragments thereof, antibodies specific to α4β7 integrin and functional fragments thereof, antibodies specific to CD3, CD4 or CD20 and functional fragments thereof, antibodies specific to interleukin 6 (IL-6), interleukin 12 (IL-12), interleukin 13 (IL-13), interleukin 23 (IL-23) or to their receptors and functional fragments thereof, antibodies specific to CXCL10/IP-10 and functional fragments thereof, and antibodies specific to p40 protein subunit and functional fragments thereof.
[9] Method according to any of the preceding items, wherein the antibody or functional fragment thereof is suitable for use in the treatment of an inflammatory bowel disease (IBD), a gastrointestinal infection, celiac disease, a colorectal cancer or a small intestine cancer, preferably an IBD, more preferably an IBD like Crohn's disease or ulcerative colitis.
[10] Method according to any of the preceding items, wherein the at least one antibody or functional fragment thereof is selected from infliximab, adalimumab, etanercept, certolizumab pegol, golimumab, visilizumab, eldelumab, abrilumab, canakinumab, tocilizumab, ustekinumab, natalizumab, etrolizumab, priliximab, vedolizumab and functional fragments thereof; from anti-TNFα antibodies or functional fragments thereof with light chain variable domains and/or heavy chain variable domains comprising complementarity-determining regions (CDRs) with amino acid sequences as disclosed in claim 2 of PCT/EP2017/056218, in claim 2 of PCT/EP2017/056246, in claim 2 of PCT/EP2017/056237 and/or in claim 2 of PCT/EP2017/056227, as originally filed; from anti-TNFα antibodies or functional fragments thereof comprising a heavy chain variable domain amino acid sequence and/or a light chain variable domain amino acid sequence according to claim 4 of PCT/EP2017/056218, claims 5 and 6 of PCT/EP2017/056246, claims 5 and 6 of PCT/EP2017/056237, and/or claim 4 of PCT/EP2017/056227, as originally filed; and combinations thereof.
[11] Method according to any of the preceding items, wherein the at least one antibody or functional fragment thereof is selected from antibodies specific to TNFα and functional fragments thereof.
[12] Method according to item 11, wherein the antibody specific to TNFα or functional fragment thereof is selected from infliximab, adalimumab, etanercept, certolizumab pegol, golimumab and functional fragments thereof; from anti-TNFα antibodies or functional fragments thereof with light chain variable domains and/or heavy chain variable domains comprising complementarity-determining regions (CDRs) with amino acid sequences as disclosed in claim 2 of PCT/EP2017/056218, in claim 2 of PCT/EP2017/056246, in claim 2 of PCT/EP2017/056237 and/or in claim 2 of PCT/EP2017/056227, as originally filed; from anti-TNFα antibodies or functional fragments thereof comprising a heavy chain variable domain amino acid sequence and/or a light chain variable domain amino acid sequence according to claim 4 of PCT/EP2017/056218, claims 5 and 6 of PCT/EP2017/056246, claims 5 and 6 of PCT/EP2017/056237, and/or claim 4 of PCT/EP2017/056227, as originally filed; and combinations thereof.
[13] Method according to any of the preceding items, wherein the buffer (buffer salt) is selected from the group consisting of acetate buffer, citrate buffer, histidine buffer, succinate buffer, phosphate buffer, hydroxymethylaminomethane (TRIS) buffer, and combinations thereof.
[14] Method according any of the preceding items, wherein the total solid content in the feed liquid (w/w) is between 0.1 and 30 wt.-%, preferably between 0.1 and 15 wt.-%, more preferably between 0.5 and 10 wt.-%, even more preferably between 1 and 10 wt.-%, even more preferably between 5 and 10 wt.-%.
[15] Method according to any of the preceding items, wherein the feed liquid comprises 5 to 95 wt.-%, preferably 75 to 90 wt.-%, more preferably 83 to 85 wt.-%, alternatively preferably e.g. 5 to 70 wt.-%, 5 to 50 wt.-%, 6 to 49 wt.-%, 15 to 45 wt.-%, 15 to 40 wt.-%, 25 to 35 wt.-%, 20 to 30 wt.-%, 30 to 40 wt.-%, 45 to 55 wt.-%, 50 to 60 wt.-%, 55 to 75 wt.-%, 60 to 70 wt.-%, 75 to 85 wt.-%, 80 to 70 wt.-%, or 85 to 95 wt.-%, of the at least one sustained release polymer, relative to the total solid content.
[16] Method according to any of the preceding items, wherein the feed liquid comprises 0.001 to 35 wt.-%, 0.01 to 30 wt.-%, preferably 0.1 to 15 wt.-%, more preferably 0.5 to 10 wt.-%, even more preferably 1 to 5 wt.-%, even more preferably about 3.5 to 4.5 wt.-%, of the at least one antibody or functional fragment thereof.
[17] Method according to any of items 1 to 15, wherein the feed liquid comprises 0.001 to 35 wt.-%, preferably 0.1 to 30 wt.-%, more preferably 1 to 25 wt.-%, even more preferably 5 to 25 wt.-%, even more preferably 10 to 25 wt.-% even more preferably 12 to 23 wt.-%, of the at least one antibody or functional fragment thereof, relative to the total solid content.
[18] Method according to any of the preceding items, wherein the feed liquid comprises 0.01-100 mg/ml, preferably 0.1-50 mg/ml, more preferably 0.5-30 mg/ml, even more preferably 1-25 mg/ml, even more preferably 5-25 mg/ml, even more preferably 5-15 mg/ml, of the at least one antibody or functional fragment thereof.
[19] Method according to any of the preceding items, wherein the feed liquid comprises a mass ratio of the at least one sustained release polymer to the at least one antibody or functional fragment thereof of between 0.1 and 200, preferably 0.5 and 100, more preferably 5 and 90, more preferably 10 and 85, even more preferably 15 and 75, even more preferably 15 and 60, even more preferably 15 and 50, even more preferably 20 and 50, even more preferably 21 and 30.
[20] Method according to any of the preceding items, wherein the mass ratio of the at least one sustained release polymer to the at least one antibody or functional fragment thereof (w/w) modifies the release rate of the at least one antibody or functional fragment thereof, such that a higher ratio results in a slower release rate.
[21] Method according to any of the preceding items, wherein the feed liquid comprises at least one filler.
[22] Method according to item 21, wherein the at least one filler is selected from the group consisting of dextrose, lactose, lactose monohydrate, lactose anhydrous, mannitol, sorbitol, xylitol, sucrose, glucose, raffinose, trehalose, dicalcium phosphate, oxides of magnesium or silicon, titanium carbonate, calcium carbonate, magnesium phosphate, porous calcium phosphate or porous magnesium phosphate, amino acids such as arginine, histidine, glycine, alanine, lysine, proline, leucine, glutamic acid, serine, aspartic acid and asparagine, and respective salts thereof, propylene glycol, polyethylene glycol, and combinations thereof; preferably dextrose, sucrose, mannitol, sorbitol, xylitol, trehalose, amino acids such as arginine, histidine, glycine, alanine, lysine, proline, leucine, glutamic acid, serine, aspartic acid and asparagine, and respective salts thereof, dicalcium phosphate, and combinations thereof; more preferably sorbitol, trehalose, sucrose, mannitol, or amino acids such as arginine, histidine, glycine, alanine, lysine, proline, leucine, glutamic acid, serine, aspartic acid and asparagine, and respective salts thereof, and combinations thereof, even more preferably trehalose, mannitol and sucrose, and combinations thereof, even more preferably trehalose or mannitol, even more preferably trehalose.
[23] Method according to any of items 21 or 22, wherein the feed liquid comprises 0.01 to 40 wt.-%, preferably 0.5 to 30 wt.-%, more preferably 2 to 20 wt.-%, of the at least one filler relative to the total amount of solids.
[24] Method according to any of the preceding items, wherein the feed liquid comprises at least one plasticizer.
[25] Method according to items 24, wherein the feed liquid comprises 0.1-30 wt.-%, preferably 0.5-20 wt.-%, more preferably about 0.5-5 wt.-%, plasticizer, relative to the total amount of the polymer solids in the feed liquid.
[26] Method according to item 24 or 25, wherein the at least one plasticizer is selected from the group consisting of triethyl citrate (TEC), dibutyl sebacate (DBS), polyethylene glycol, acetyl triethyl citrate, butyl citrate, polysorbates, 1, 2-propylene glycol, and combinations thereof, preferably TEC or DBS.
[27] Method according to any of items 24 to 26, wherein the at least one plasticizer is DBS.
[28] Method according to any of the preceding items, wherein the feed liquid further comprises a pore forming agent.
[29] Method according to item 28, wherein the pore forming agent is hypromellose, methylcellulose, hydroxypropyl cellulose, carboxymethyl cellulose, polyvinylpirrolidone, or polyvinyl alcohol-polyethylene glycol (PVA-PEG) graft copolymer, and combinations thereof.
[30] Method according to any of the preceding items, wherein the feed liquid further comprises a viscosity-reducing agent, preferably arginine succinate, arginine glutamate or arginine hydrochloride.
[31] Method according to any of the preceding items, wherein the feed liquid further comprises a surfactant.
[32] Method according to item 31, wherein the surfactant is selected from polysorbates such as polysorbates 20, 28, 40, 60, 65, 80, 81 and 85; poloxamers such as poloxamers 124, 181, 188, 237, 331, 338 and 407, glyceryl monostearate, polyethoxylated castor oil, PEG-40 hydrogenated castor oil, macrogol 15 hydroxystearate, polyoxyl 15 hydroxystearate, caprylocaproyl macrogol-8 glyceride, D-α-tocopherol polyethylene glycol 1000 succinate, glyceryl monostearate, lecithin, sorbitan monopalmitate, cetyl alcohol, oleyl alcohol, sodium glycolate, sodium de(s)oxycholate, block copolymer of polyethylene and polypropylene glycol, polyethylene glycol, polypropylene glycol, alkyl poly(ethylene oxide), alkyl glycoside, alkyl polyglucoside, octyl glucoside, decyl maltoside, and combinations thereof, and combinations thereof.
[33] Method according to item 31 or 32, wherein the feed liquid comprise 0.01 to 2.0 wt.-%, preferably 0.05 to 1 wt.-%, more preferably about 0.1 to 0.5 wt.-% surfactant.
[34] Method according to any of the preceding items, wherein the feed liquid comprises at least one further excipient selected from divalent cations (e.g., Ca2+, Zn2+, Mg2+, and Mn2+), antioxidants, humectants, protective colloids, dyes, and combinations thereof.
[35] Method according to any of the preceding items, wherein the feed liquid is an aqueous suspension where 100 % of the solvent used is water.
[36] Method according to any of the preceding items, wherein during step a) the at least one antibody or functional fragment thereof in the form of an aqueous solution is added to the feed liquid, preferably already comprising the at least one sustained release polymer.
[37] Method according to any of items 1 to 35, wherein during step a) the at least one antibody or functional fragment thereof in the form of a powder is added to the feed liquid, preferably already comprising the at least one sustained release polymer.
[38] Method according to any of the preceding items, wherein during step b) the feed liquid is sprayed at a spray rate of between 0.5 and 100 g/min, preferably 1 and 70 g/min, more preferably 10 and 60 g/min, most preferably 15 and 55 g/min.
[39] Method according to any one of items 1 to 37, wherein the feed liquid is sprayed at a spray rate of 0.1-10 g/min, preferably 0.5-5 g/min, more preferably 1.5-4.5 g/min, even more preferably 2-3 g/min.
[40] Method according to any of the preceding items, wherein the at least one antibody or functional fragment thereof is dissolved in the feed liquid of step a).
[41] Method according to any of the preceding, wherein the nozzle is a bi-fluid nozzle or an ultrasonic nozzle, preferably a bi-fluid nozzle, alternatively preferably an ultrasonic nozzle.
[42] Method according to item 41, wherein the nozzle is a bi-fluid nozzle, with a diameter of 0.1-2 mm, preferably 0.2-0.1 mm, more preferably 0.4 to 0.8 mm, most preferably about 0.6 mm.
[43] Method according to item 41 or 42, wherein the nozzle is a bi-fluid nozzle and the nozzle gas pressure is lower than 200 kPa, preferably lower than 100 kPa.
[44] Method according to item 41, wherein the nozzle is an ultrasonic nozzle, which is operated at a frequency of 20 to 120 kHz, preferably 40 to 80 kHz, more preferably about 60 kHz.
[45] Method according to any of the preceding items, wherein during step b) the inlet air flow of the drying gas is between 0.2 and 5 m³/min, preferably 0.2 and 3 m³/min, more preferably 0.3 and 2.5 m³/min.
[46] Method according to any of the preceding items, wherein the drying gas in step b) is selected from the group consisting of air, nitrogen, helium, carbon dioxide, xenon, preferably air or nitrogen.
[47] Method according to any of the preceding items, wherein during step b) the inlet temperature of the drying gas is between 50 °C and 200 °C, preferably between 80°C and 190°C, more preferably between 90 °C and 170 °C, even more preferably between 90 °C and 150 °C, even more preferably between 100 °C and 140 °C.
[48] Method according to any of the preceding items, wherein during step b) the outlet temperature of the drying gas with the particles dispersed therein is between 25 °C and 120 °C, preferably between 40°C and 100°C, more preferably between 45 °C and 90 °C, even more preferably between 45 °C and 80 °C, even more preferably between 50 °C and 75 °C.
[49] Method according to any of the preceding items, wherein during step d), the particle concentration means separates the particles from the drying gas by removing between 50 vol.-% to 100 vol.-% of the drying gas, preferably 60 vol.-% to 100 vol.-%, more preferably 70 vol.-% to 100 vol.-%, even more preferably at least 80 vol.-%, 90 vol.-%, 95 vol.-%, 97 vol.-% or 99 vol.-%.
[50] Method according to any of the preceding items, wherein the particle concentration means in step d) comprises a cyclone separator.
[51] Method according to item 50, wherein during step d) the temperature of the drying gas with the particles dispersed therein before the cyclone is between 10 °C and 70 °C, preferably 15°C and 60°C, more preferably 20 °C and 55 °C, even more preferably 25 °C and 50 °C.
[52] Method according to any of the preceding items, wherein the recovery is at least 50%, preferably at least 70 %, more preferably at least 80%, even more preferably at least 85%, even more preferably at least 90 %.
[53] Method according to any of the preceding items, wherein the particles after step d) have a particle-size distribution (PSD) D50 of 0.5-200 µm, preferably 1-100 µm, more preferably 5-75 µm, even more preferably 5-50 µm, even more preferably 10-40 µm, even more preferably about 10-30 µm.
[54] Method according to any of the preceding items, wherein the particles after step d) have a particle-size distribution such that at least 90 % of the particles have a particle size of 0.5-200 µm, preferably 1-120 µm, more preferably 5-100 µm, even more preferably 10-90 µm, even more preferably 20-80 µm, even more preferably 30-70 µm.
[55] Method according to any of the preceding items, wherein after step d), the residual moisture in the particles is less than 15 wt.-%, preferably less than 10 wt.-%, e.g. less than 9 wt.-%, 8 wt.-%, 7 wt.-%, 6 wt.-%, 5 wt.-%, 4 wt.%, 3 wt.-%, 2 wt.-%, or 1 wt.-%, relative to the total weight of the particle after step d).
[56] Method according to any of the preceding items, wherein the particles comprise 0.01 to 50 wt.-%, preferably 0.1 to 30 wt.-%, more preferably 0.5 to 25 wt.-%, even more preferably 0.5 to 20 wt.-%, even more preferably 0.5 to 10 wt.-%, even more preferably 1 to 7 wt.-% of the at least one antibody or functional fragment thereof, relative to the total weight of the particles after step d).
[57] Method according to any of the preceding items, wherein the particles, upon reconstitution (immersion) in an aqueous solution, give rise to a sustained release profile (sustained release particles).
[58] Method according to item 57, wherein the sustained release particles are part of a solid dosage form.
[59] Method according to any of the preceding items, wherein the particles are intended for oral or rectal administration.
[60] Method according to any of the preceding items, wherein the particles are intended for oral administration.
[61] Method according to any one of the preceding items, further comprising, after step d), the step of applying at least one additional coating in the form of a delayed release coating.
[62] Method according to any one of the preceding items, wherein each particle has a predetermined axis and the same predetermined cross-sectional profile, such that at least 50 %, at least 80 % of the particles, preferably 90 %, more preferably 95 %, by number, have a median aspect ratio between 0.6 and 1.8, the aspect ratio being defined as particle length along the predetermined axis divided by the smallest cross-sectional dimension.
[63] Method according to item 62, wherein the median aspect ratio is above 0.7 preferably above 0.8, more preferably above 0.9, and below 1.7, preferably 1.6, more preferably below 1.5, more preferably 1.4, even more preferably below 1.3, even more preferably below 1.2, most preferably about 1.
[64] Method according to item 62 or 63, wherein the particles have a span of aspect ratio of less than 0.9, preferably less than 0.8, more preferably less than 0.7, even more preferably less than 0.6, most preferably less than 0.5.
[65] Method according to any of the preceding items, comprising after step d) the step of preparing a multiparticulate solid dosage form, e.g. a sachet, stick pack, tablet, mini tablet, pellet, bead, sphere, spheroid, mini sphere, straw device (X-straw) or capsule, comprising a plurality of particles.
[66] Method according to item 65, wherein the multiparticulate solid dosage form, e.g. sachet, stick pack, tablet, mini tablet, pellet, bead, sphere, spheroid, mini sphere, straw device (X-straw®) or capsule, comprises a total amount of the at least one antibody or functional fragment thereof prepared into particles suitable for oral administration to a human patient.
[67] Method according to item 65 or 66, wherein the multiparticulate solid dosage form (e.g. sachet, stick pack, tablet, mini tablet, pellet, bead, sphere, spheroid, mini sphere, straw device (X-straw®) or capsule), comprises a therapeutically effective dose of the at least one antibody or functional fragment thereof suitable for oral administration to a human patient.
[68] Method according to any of items 65 to 67, wherein the multiparticulate solid dosage form is a tablet, mini-tablet, pellet, sphere or mini sphere, prepared by direct compression, extrusion-spheronization or encapsulation, preferably for oral administration.
[69] Method according to item 65 to 68, wherein the multiparticulate solid dosage form is intended for oral administration.
[70] Method according to any of items 65 to 69, comprising the further step of coating the multiparticulate solid dosage form with a delayed release coating.
[71] Method according to item 61 or 70, wherein the delayed release coating is applied by spray coating, preferably fluidized-bed spray coating.
[72] Method according to item 61, 70 or 71, wherein the delayed release coating comprises at least one component selected from coating materials that disintegrate pH-dependently; coating materials that disintegrate time-dependently; coating materials that disintegrate due to enzymatic triggers in the intestinal environment; and combinations thereof.
[73] Method according to item 72, wherein
   - the coating materials that disintegrate pH-dependently are selected from poly vinyl acetate phthalate; cellulose acetate trimellitate; hydroxypropyl methylcellulose phthalate HP-50, HP-55 or HP-55S; cellulose acetate phthalate; hydroxypropyl methylcellulose acetate succinate (HPMCAS); poly(methacrylic acid, ethyl acrylate) 1:1 (Eudragit® L100-55, Eudragit® L30D-55); poly(methacrylic acid, methyl methacrylate) 1:1 (Eudragit® L-100, Eudragit® L12.5); poly(methacrylic acid, methyl methacrylate) 1:2 (Eudragit® S-100, Eudragit® S12,5, Eudragit® FS30D), and combinations thereof;
   - the coating materials that disintegrate time-dependently are selected from poly(ethyl acrylate, methyl methacrylate) 2 : 1 (e.g. Eudragit® NM 30D, Eudragit® NE 30D); poly(ethyl acrylate, methyl methacrylate, methacrylic acid 7 : 3 : 1 (e.g. Eudragit® RS 30D); ethylcellulose (e.g. Surelease® or Aquacoat ECD); poly(ethyl acrylate, methyl methacrylate, trimethylammonioethyl methacrylate chloride) 1 : 2 : 0.2 (e.g. Eudragit® RL 30D); polyvinyl acetate (eg. Kollicoat® SR 30D); and combinations thereof; and
   - the coating materials that disintegrate due to enzymatic triggers in the intestinal environment are selected from chondroitin sulfate; cyclodextrin; pectin; guar gum; chitosan; inulin; lactulose; raffinose; stachyose; alginate; dextran; xanthan gum; locust bean gum; arabinogalactan; amylose; pullulan; carrageenan; scleroglucan; chitin; curdulan; levan; amylopectin; starch; resistant starch; azo compounds being degraded by azo bonds splitting bacteria; and combinations thereof.
[74] Method according to item 72 or 73, wherein the delayed release coating is comprises a combination of at least one coating material that disintegrates pH-dependently and at least one coating material that disintegrates due to enzymatic triggers in the intestinal environment.
[75] Method according to any one of items 72 to 74, wherein the delayed release coating comprises a combination of at least one pH sensitive (enteric) polymer, preferably poly(methacrylic acid, methyl methacrylate) 1:2 (e.g. Eudragit® S), and at least one polysaccharide selected from chondroitin sulfate, cyclodextrin, chitosan, dextran, arabinogalactan, amylose, pullulan, carrageenan, scleroglucan, chitin, curdulan, levan, amylopectin, starch, resistant starch, azo compounds being degraded by azo bonds splitting bacteria, and combinations thereof, preferably resistant starch.
[76] Method according to any one of items 72 to 75, wherein the delayed release coating comprises i) an inner coating comprising a partially neutralized enteric polymer adjusted to pH 8, e.g. partially neutralized poly(methacrylic acid, methyl methacrylate 1:2 (Eudragit® S) adjusted to pH 8, and containing a buffer salt, and ii) an outer coating comprising a combination of at least one enteric polymer, preferably poly(methacrylic acid, methyl methacrylate) 1:2 (Eudragit® S), and at least one polysaccharide selected from chondroitin sulfate, cyclodextrin, chitosan, dextran, arabinogalactan, amylose, pullulan, carrageenan, scleroglucan, chitin, curdulan, levan, amylopectin, starch, resistant starch, azo compounds being degraded by azo bonds splitting bacteria, and combinations thereof, preferably resistant starch.
[77] Method according item 72 or 76, wherein the at least one component, e.g. the combination of the at least one enteric polymer, preferably poly(methacrylic acid, methyl methacrylate) 1:2 (e.g. Eudragit® S), and the at least one polysaccharide, preferably resistant starch, is dispersed in an organic solvent, a mixture of organic solvents or a mixture of at least one organic solvent and water, which is applied to the multiparticulate solid dosage form or the particles, preferably by spray coating, more preferably fluidized-bed spray coating.
[78] Method according item 77, wherein the combination is dispersed in a mixture of at least one organic solvent and water, prepared by mixing a enteric polymer dissolved in an organic solvent with an aqueous re-dispersion of the at least one polysaccharide, or wherein the combination is dispersed in a mixture of at least one organic solvent and water, prepared by mixing an aqueous dispersion of the at least one enteric polymer with an aqueous dispersion of the at least one polysaccharide comprising residual organic solvent.
[79] Method according to any one of items 72 to 78, comprising a delayed release coating for targeted release of the at least one antibody or functional fragment thereof starting in the terminal ileum, the ileocolonic region, the ascending colon, transverse colon or the descending colon.
[80] Method according to any of the preceding items, wherein the particles or the multiparticulate solid dosage form allow(s) the recovery of at least 40%, preferably at least 50%, more preferably at least 60 %, even more preferably at least 70 %, even more preferably at least 80 %, even more preferably at least 85%, even more preferably at least 90%, even more preferably at least 93%, most preferably at least 95 %, of the at least one antibody or functional fragment thereof from the drug coating within 4 h, or 6 h, or 8 h, or 10 h, or 12 h, or 14 h, or 16 h, or 18 h, or 20 h, or 22 h, or 24 h, or 26 h, or 28 h, or 30 h, etc., of continuously immersing the particles or the multiparticulate solid dosage form in an aqueous solution under continuous agitation.
[81] Method according to any of the preceding items, wherein the at least one sustained release polymer in the particles, gives rise to a release profile for the at least one antibody or functional fragment thereof, where a sustained release with a substantially constant release rate of at least 80 %, preferably at least 90 %, of the at least one antibody or fragment thereof in the particles is achieved over 4-32 h, preferably 8-30 h, more preferably 10-28 h, e.g. 10 h, 12h, 14 h, or 16 h, or 18h, or 20 h, or 22 h, or 24 h, or 26 h, or 28 h, upon continuously immersing the particles or the multiparticulate solid dosage form in an aqueous solution, under continuous agitation.
[82] Method according to any of the preceding items, wherein the at least one sustained release polymer in the particles, allows a sustained release of the at least one antibody or functional fragment thereof over a time period of at least 5 h, preferably at least 10 h, more preferably at least 15 h, even more preferably at least 20 h, most preferably at least 24 h, upon continuously immersing the particles or multiparticulate solid dosage form in an aqueous solution under continuous agitation.
[83] A method for preparing a multiparticulate solid dosage form, comprising the steps of i) preparing particles comprising at least one antibody or functional fragment thereof as active agent, a buffer and at least one sustained release polymer, and optionally a plasticizer and/or a surfactant and/or filler, by spray drying, and ii) preparing a sachet, stick pack, straw device (X-straw®), tablet, mini tablet, pellet, bead, sphere, mini sphere, or capsule comprising a plurality of particles, preferably for oral administration, and optionally iii) coating the multiparticulate solid dosage form with a delayed release coating.
[84] Method according to item 83, wherein each particle has a predetermined axis and the same predetermined cross-sectional profile, such that at least 50 %, at least 80 % of the particles, preferably 90 %, more preferably 95 %, by number, have a median aspect ratio between 0.6 and 1.8, the aspect ratio being defined as particle length along the predetermined axis divided by the smallest cross-sectional dimension.
[85] Method according to item 83 or 84, wherein the median aspect ratio is above 0.7 preferably above 0.8, more preferably above 0.9, and below 1.7, preferably 1.6, more preferably below 1.5, more preferably 1.4, even more preferably below 1.3, even more preferably below 1.2, most preferably about 1.
[86] Method according to any of items 83 to 85, wherein the particles are prepared by spray drying according to the method of any of items 1 to 64.
[87] Method according to any of items 83 to 86, wherein the multiparticulate solid dosage form is a tablet, capsules, mini-tablet, pellet, sphere or mini sphere, prepared by direct compression, encapsulation or extrusion-spheronization, preferably for oral administration.
[88] Method according to any of the preceding items wherein the fraction of total content of antibody or functional fragment thereof present in the particles or in the multiparticulate solid dosage form as dimers and other aggregates does not exceed by more than 20 %, preferably 15%, more preferably 10 %, even more preferably 7 %, even more preferably 8%, even more preferably 5 %, 3 %, 2 % or 1.5 %, the fraction of total antibody or functional fragment thereof present as dimers and other aggregates at the time of adding the antibody or functional fragment thereof to the feed liquid.
[89] Method according to any of the preceding items wherein the fraction of total content of antibody or functional fragment thereof present in the particles or in the multiparticulate solid dosage form as fragments of the full-length antibody or functional fragment thereof does not increase substantially compared to the time of adding the antibody or functional fragment thereof to the feed liquid.
[90] Method according to any of the preceding items wherein the fraction of total content of antibody or functional fragment thereof present in the particles or in the multiparticulate solid dosage form as fragments of the full-length antibody or functional fragment thereof does not exceed by more than 20 %, preferably 15 %, more preferably 12 %, even more preferably 10 %, even more preferably 8%, even more preferably 6 %, 5 %,3 %, 2 %, or 1.5 %, the fraction of total content of antibody or functional fragment thereof present as fragments of the full-length antibody or functional fragment thereof at the time of adding the antibody or functional fragment thereof to the feed liquid.
[91] A particle obtainable by the method of any one of items 1 to 90.
[92] A multiparticulate solid dosage form obtainable by the method of any one of items 64 to 90.
[93] Particle or multiparticulate solid dosage form according to item 91 or 92 for use in the topical treatment of a gastrointestinal disease, preferably IBD, celiac disease, a gastrointestinal infection, a colorectal cancer or a small intestine cancer, more preferably an IBD.
[94] Particle or multiparticulate solid dosage form for use according to item 93, wherein the IBD is Crohn's disease or ulcerative colitis.
[95] Particle or multiparticulate solid dosage form according to any one of items 91 to 94 for use in the topical treatment in the terminal ileum, the ileocolonic region, the ascending colon, transverse colon or the descending colon of a patient.
[96] A particle, comprising at least one antibody or functional fragment thereof as active agent, a buffer and at least one sustained release polymer, and optionally a plasticizer and/or a surfactant and/or filler, obtainable by spray drying.
[97] Particle according to item 96, obtainable by spray drying comprising the steps of
   a) preparing a feed liquid comprising the at least one antibody or functional fragment thereof, the buffer and the at least one sustained release polymer, and optionally a plasticizer and/or a surfactant and/or filler, all in an aqueous suspension;
   b) spraying the feed liquid through a nozzle into a primary drying chamber to form droplets, wherein the droplets are dispersed in a drying gas;
   c) drying the droplets, by evaporating at least a portion of the liquid in the droplets into the drying gas, to form particles dispersed in the drying gas; and
   d) directing the drying gas with the particles dispersed therein, through a particle concentration means to recover the particles.
[98] A multiparticulate solid dosage form, comprising a plurality of particles, each of the particles obtainable by the method of any one of items 1 to 64, wherein the multiparticulate solid dosage form preferably is a sachet, stick pack, tablet, mini tablet, pellet, bead, sphere, spheroid, mini sphere, straw device (X-straw®), or capsule).
[99] A multiparticulate solid dosage form, comprising a plurality of particles, wherein each particle comprises at least one antibody or functional fragment thereof as active agent, a buffer and at least one sustained release polymer, and optionally a plasticizer and/or a surfactant and/or filler, and is obtainable by spray drying.
[100] Multiparticulate solid dosage form according to item 99, wherein the particles are obtainable by spray drying comprising the steps of
   a) preparing a feed liquid comprising the at least one antibody or functional fragment thereof, the buffer and the at least one sustained release polymer, all in an aqueous suspension;
   b) spraying the feed liquid through a nozzle into a primary drying chamber to form droplets, wherein the droplets are dispersed in a drying gas;
   c) drying the droplets, by evaporating at least a portion of the liquid in the droplets into the drying gas, to form particles dispersed in the drying gas; and
   d) directing the drying gas with the particles dispersed therein, through a particle concentration means to recover the particles.
[101] Particle or multiparticulate solid dosage form according to any one of items 96 to 100 for use in the topical treatment of a gastrointestinal disease, preferably IBD, celiac disease, a gastrointestinal infection, a colorectal cancer or a small intestine cancer, more preferably an IBD
[102] Particle or multiparticulate solid dosage form according to any one of items 96 to 101, wherein the at least one antibody or functional fragment thereof is as defined in any one of items 7-12 and 56; and/or wherein the buffer is as defined in item 13 or 14; and/or wherein the at least one sustained release polymer is as defined in any one of items 2-4, and/or wherein the optional plasticizer and/or surfactant and/or filler is/are as defined in any one of items 21 to 34.
[103] Particle or multiparticulate solid dosage form according to any one of items 96 to 102, wherein the particles have the properties as defined in any of items 19, 53-55, and 58 to 64.
[104] Particle or multiparticulate solid dosage form according to any one of items 97 to 103, allowing the recovery of at least 80 %, preferably at least 85 %, more preferably at least 93 %, even more preferably at least 95 %, even more preferably at least 97%, even more preferably at least 98%, of the at least one antibody or functional fragment thereof from the particles.
[105] Particle or multiparticulate solid dosage form according to any one of items 96 to 104, allowing the recovery of at least 80 %, preferably at least 85 %, more preferably at least 93 %, even more preferably at least 95 %, even more preferably at least 97%, even more preferably at least 98%, of the at least one antibody or functional fragment thereof from the particles, within 4 h, or 6 h, or 8 h, or 10 h, or 12 h, or 14 h, or 16 h, or 18 h, or 20 h, or 22 h, or 24 h, or 26 h, or 28 h, or 30 h, or 32 h, or 34 h, or 36 h, etc., of continuously immersing the particles or the multiparticulate solid dosage form in an aqueous solution under continuous agitation (sustained release).
[106] Multiparticulate solid dosage form according to any one of items 98 to 105, wherein at least 80% by number of those particles, preferably 90%, more preferably 95%, have a median aspect ratio between 0.6 and 1.8, the aspect ratio being defined as particle length along the predetermined axis divided by the smallest cross-sectional dimension.
[107] Method according to item 106, wherein the median aspect ratio is above 0.7 preferably above 0.8, more preferably above 0.9, and below 1.7, preferably 1.6, more preferably below 1.5, more preferably 1.4, even more preferably below 1.3, even more preferably below 1.2, most preferably about 1.
[108] Method according to item 106 or 107, wherein the particles have a span of aspect ratio of less than 0.9, preferably less than 0.8, more preferably less than 0.7, even more preferably less than 0.6, most preferably less than 0.5.
[109] Multiparticulate solid dosage form according to any of items 98 to 108, wherein the multiparticulate solid dosage form is prepared by compression, encapsulation or extrusion-spheronization.
[110] Multiparticulate solid dosage form according to any of items 106 to 109, wherein the particles are prepared by spray drying according to the method of any of any one of items 1 to 65.
[111] Multiparticulate solid dosage form according to any of items 106 to 110, wherein the multiparticulate solid dosage form further comprises a delayed release coating, which is applied as a further coating.

### BRIEF DESCRIPTION OF THE DRAWINGS

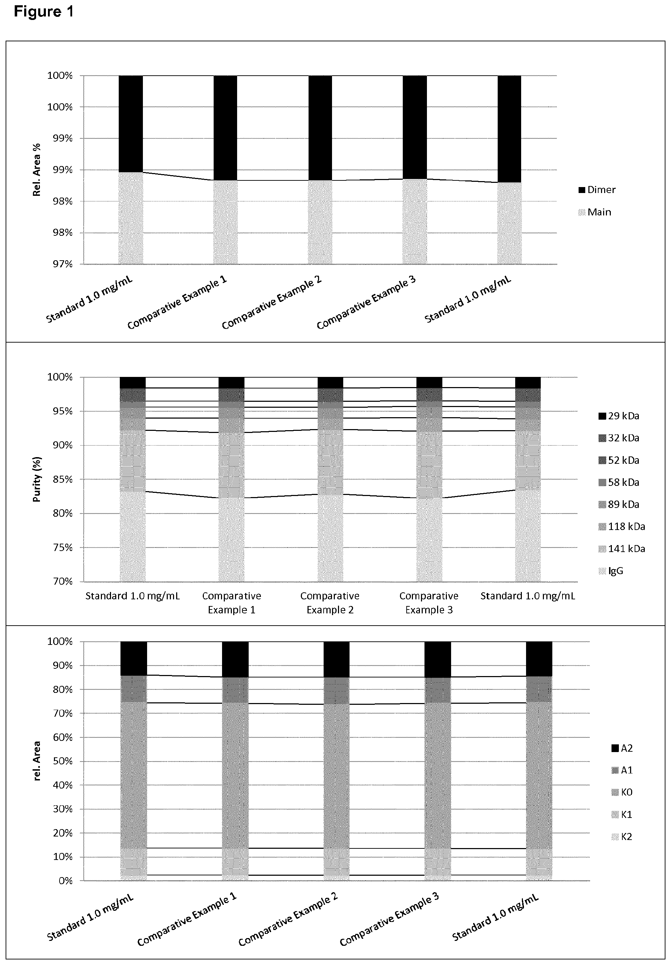

Figure 1: shows the relative dimer to monomer content (determined by HPLC-SEC) (A), relative fragmentation profile (determined by microchip electrophoresis) (B) and acidic/basic species (surface net charge) profile of adalimumab reconstituted from spray-dried adalimumab processed with an inlet air temperature of 100°C (Comparative Example 1 and Comparative Example 2) or 120°C (Comparative Example 3) in citrate-TRIS buffer pH 7.

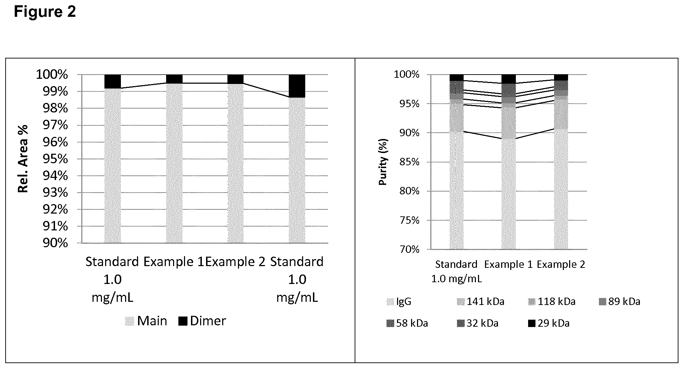

Figure 2: shows the relative dimer to monomer content (determined by HPLC-SEC) and the relative fragmentation profile (determined by microchip electrophoresis) of adalimumab released in citrate-TRIS buffer pH from particles obtained by spray-drying, comprising adalimumab and Eudragit® RS 300 in (polymer:adalimumab mass ratio of 21.9) using a feed solution containing 18.8 % wt total solids. The feed suspension was processed with an inlet air temperature of 100°C (Example 1) or 120 °C (Example 2).

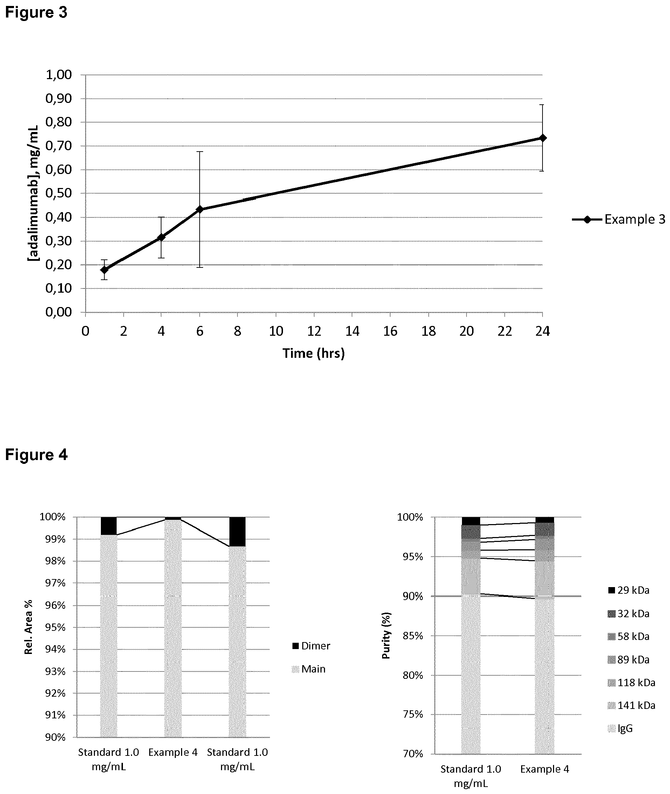

Figure 3: shows the adalimumab release from particles obtained by spray-drying, comprising adalimumab and Eudragit® RS 30D in (polymer:adalimumab mass ratio of 21.9) using a feed solution containing 9.3 % wt total solids in citrate-TRIS pH 7 dissolution buffer (Example 3). Average and standard deviation of 3 measurements are shown.
Figure 4: shows the relative dimer to monomer content (determined by HPLC-SEC) and the relative fragmentation profile (determined by microchip electrophoresis) of adalimumab recovered in citrate-TRIS buffer pH 7 from particles obtained by spray-drying, comprising adalimumab and Kollicoat® SR 30D (polymer:adalimumab mass ratio of 89.7) using a feed solution containing 29.4 % wt total solids (Example 4). The feed suspension was processed with an inlet air temperature of 120° and nozzle air of 65 l/min

### DETAILED DESCRIPTION

The present invention relates to a method for preparing particles comprising at least one antibody or functional fragment thereof as active agent, a buffer and at least one sustained release polymer, and optionally a surfactant and/or a plasticizer and/or a filler, by spray drying; the method comprising the steps of a) preparing a feed liquid comprising the at least one antibody or functional fragment thereof, the buffer and the at least one sustained release polymer, all in an aqueous suspension; b) spraying the feed liquid through a nozzle into a primary drying chamber to form droplets, wherein the droplets are dispersed in a drying gas; c) drying the droplets, by evaporating at least a portion of the liquid in the droplets into the drying gas, to form particles dispersed in the drying gas; and d) directing the drying gas with the particles dispersed therein, through a particle concentration means to recover the particles.

The term "particle" as used herein is general in meaning and is understood to mean small discrete object, small discrete unit, or small discrete portion, and encompasses a grain, powder grain, granule and the like. Preferably, a particle is a solid unit, and is preferably generally spherical or spheroidal in shape. The term "multiparticulate solid dosage form" as used herein is understood to mean "comprising a plurality of particles/small discrete units/small discrete portions/small discrete objects" and encompasses the term "multiparticulate ". The term "multiparticulate solid dosage form" as used herein is understood to include e.g. sachets, stick packs, straw devices (X-straw®), tablets, mini tablets, pellets, beads, caplets, pills, core, spheroids, spheres, mini spheres, granule, capsules, solid dosage form, multi unit pellet system, and the like. The term "solid dosage form" encompasses the terms "multiparticulate" and "multiparticulate solid dosage form". The term "solid dosage form" as used herein may be understood to be equivalent to "solid pharmaceutical dosage form" or "pharmaceutical composition formulated into a solid dosage form". The term "solid dosage form" includes for example sachets, stick packs, straw devices, pellets, capsules, caplets, pills, cores, beads, spheroids spheres, mini spheres, granules, tablets, mini tablets and the like.. Multiple particles of the present invention may be combined into a single-unit formulation in the form of a multiparticulate or multiparticulate solid dosage form, for example a tablet, mini-tablet, spheroid, sphere, mini sphere, capsule, granule, beads core, sachet, stick pack, straw device (Xstraw®), caplet, or pill.

The term "about", as used herein, indicates the value or range of a given quantity can include quantities ranging within 10% of the stated value or range, or optionally within 5% of the value or range, or in some embodiments within 1% of the value or range.

Generally, unless otherwise indicated, "wt.-%" as used herein, refers to the weight percent of a substance or component relative to the total weight of a whole, e.g. the feed liquid, particle, solid dosage form, total solid content, etc. Generally, unless otherwise indicated, "vol.-%" as used herein, refers to the volume percent of a liquid or gas, relative to the relative to the total volume of a whole.

The particle prepared by the present invention comprises at least one antibody or functional fragment thereof as active agent. The term "antibody", in the context of the present invention, refers to "immunoglobulin" (Ig), which is defined as a protein belonging to the class IgG, IgM, IgE, IgA, or IgD (or any subclass thereof), and includes all conventionally known antibodies and functional fragments thereof. The at least one antibody or functional fragment thereof used for the present invention is an active agent, i.e. the at least one antibody or functional fragment thereof is incorporated into the particle or the multiparticulate solid dosage form due to the pharmacological activity of the antibody or functional fragment thereof in a patient.

In the context of the present invention, a "functional fragment" of an antibody/immunoglobulin is defined as antigen-binding fragment or other derivative of a parental antibody that essentially maintains the properties of such a parental antibody. An "antigen-binding fragment" of an antibody/immunoglobulin is defined as a fragment (e.g., a variable region of an IgG) that retains the antigen-binding region. An "antigen-binding region" of an antibody typically is found in one or more hypervariable region(s) of an antibody, i.e., the CDR-1, -2, and/or -3 regions. "Antigen-binding fragments" according to the invention include the domain of a F(ab')₂ fragment and a Fab fragment. "Functional fragments" of the invention include Fab fragment, F(ab')₂ fragment, Fab' fragment, scFv, dsFv, VHH, diabody, triabody, tetrabody, Fc fusion protein and minibody. The F(ab')₂ or Fab domain may be engineered to minimize or completely remove the intermolecular disulphide interactions that occur between the CH1 and CL domains. The antibodies or functional fragments thereof used for the present invention may be part of bi- or multifunctional constructs.

Fab fragments can be obtained as the purified digestion products after digestion of an antibody with a cysteine proteinase like papain (EC 3.4.22.2). F(ab')₂ fragments can be obtained as the purified digestion products after digestion of an antibody with pepsin (EC 3.4.23.1) or IdeS (Immunoglobulin degrading enzyme from Streptococcus pyogenes; EC 3.4.22). Fab' fragments can be obtained from F(ab')₂ fragments in mild reducing conditions, whereby each F(ab')₂ molecule gives rise to two Fab' fragments. An scFv is a single chain Fv fragment in which the variable light ("V_{L}") and variable heavy ("V_{H}") domains are linked by a peptide bridge.

A "diabody" is a dimer consisting of two fragments, each having variable regions joined together via a linker or the like (hereinafter referred to as diabody-forming fragments), and typically contain two V_{L}s and two V_{H}s. Diabody-forming fragments include those consisting of V_{L} and V_{H}, V_{L} and V_{L}, V_{H} and V_{H}, etc., preferably V_{H} and V_{L}. In diabody-forming fragments, the linker joining variable regions is not specifically limited, but preferably short enough to avoid noncovalent bonds between variable regions in the same fragment. The length of such a linker can be determined as appropriate by those skilled in the art, but typically 2-14 amino acids, preferably 3-9 amino acids, especially 4-6 amino acids are used. In this case, the V_{L} and V_{H} encoded on the same fragment are joined via a linker short enough to avoid noncovalent bonds between the V_{L} and V_{H} on the same chain and to avoid the formation of single-chain variable region fragments so that dimers with another fragment can be formed. The dimers can be formed via either covalent or noncovalent bonds or both between diabody-forming fragments.

Moreover, diabody-forming fragments can be joined via a linker or the like to form single-chain diabodies (sc(Fv)₂). By joining diabody-forming fragments using a long linker of about 15-20 amino acids, noncovalent bonds can be formed between diabody-forming fragments existing on the same chain to form dimers. Based on the same principle as for preparing diabodies, polymerized antibodies such as trimers or tetramers can also be prepared by joining three or more diabody-forming fragments.

In one embodiment, the functional antibody fragment in the particle prepared by the inventive method is a Fab fragment, a F(ab')2 fragment, a Fab' fragment, an scFv, a dsFv, a VHH, a diabody, a triabody, a tetrabody, an Fc fusion protein or a minibody. Preferred functional fragments used in the present invention are Fab fragments, F(ab')₂ fragments, Fab' fragments, scFv and diabodies.

The antibody or functional fragment thereof used in the inventive method for the preparation of the particle is not particularly limited. In one embodiment, the antibody or functional fragment thereof is an antibody. In another embodiment of the present invention, the antibody or functional fragment thereof is functional fragment as defined above. The antibody or functional fragment thereof may further comprise one or more modifications, e.g. in the form of added or substituted residues, that improve stability, specificity or targeting. These may include any such modifications that are known in the art.

The antigen against which the antibody or functional fragment is directed i.e. the immunogen, peptide, protein, or other molecular structure to which the antibody or functional fragment thereof can specifically bind, is not limited. In its most general form (and when no defined reference is mentioned), "specific to" or "specific binding" refers to the ability of the antibody or functional fragment thereof to discriminate between the target of interest and an unrelated biomolecule (e.g. for antibodies specific to human TNFα to discriminate between human TNFα and an unrelated biomolecule), as determined, for example, in accordance with specificity assay methods known in the art. Such methods comprise, but are not limited to, Western blots and enzyme-linked immunosorbent assay (ELISA) tests. For example, a standard ELISA assay can be carried out. Typically, determination of binding specificity is performed by using not a single reference biomolecule, but a set of about three to five unrelated biomolecules, such as milk powder, BSA, transferrin or the like. In one embodiment of the present invention the antibody or functional fragment thereof is suitable for use in the treatment of a gastrointestinal disease, preferably an inflammatory bowel disease (IBD), cancer (such as colorectal cancer or small intestine cancer), celiac disease, an infection (such as Clostridium difficile infection), more preferably an IBD e.g. Crohn's disease or ulcerative colitis. In another embodiment of the present invention the antibody or functional fragment thereof is suitable for use in the topical treatment in the ileum or large intestine of the gastrointestinal tract of a patient.

In a further embodiment of the present invention the antibody or functional fragment thereof is selected from antibodies specific to tumor necrosis factor alpha (TNFα) and functional fragments thereof, antibodies specific to α4β7 integrin and functional fragments thereof, antibodies specific to CD3, CD4 or CD20 and functional fragments thereof, antibodies specific to interleukin 6 (IL-6), interleukin 12 (IL-12), interleukin 13 (IL-13), interleukin 23 (IL-23) or to their receptors and functional fragments thereof, antibodies specific to CXCL10/IP-10 and functional fragments thereof, and antibodies specific to p40 protein subunit and functional fragments thereof. In yet another embodiment of the present invention the antibody or functional fragment thereof is selected from infliximab, adalimumab, etanercept, certolizumab pegol, golimumab, visilizumab, eldelumab, abrilumab, canakinumab, tocilizumab, ustekinumab, natalizumab, etrolizumab, priliximab, vedolizumab and functional fragments thereof.

In one embodiment of the present invention, the at least one antibody or functional fragment thereof in the particle prepared by the inventive method specifically binds to TNFα. The terms "anti-TNFα antibody", "TNFα antibody" and "antibody specific to TNFα" as used herein are interchangeable. In one embodiment, specific binding refers to the ability of the antibody or fragment to discriminate between human TNFα and human TNFβ. In a preferred embodiment of the present invention the TNFα antibody or functional fragment thereof is a TNFα antibody. In an alternatively preferred embodiment of the present invention the TNFα antibody or functional fragment thereof is a functional fragment of a TNFα antibody.

Several monoclonal antibodies against TNFα have been described in the prior art. Meager et al. (Hybridoma, 6, 305-311, 1987) describe murine monoclonal antibodies against recombinant TNFα. Fendly et al. (Hybridoma, 6, 359-370, 1987) describe the use of murine monoclonal antibodies against recombinant TNFα in defining neutralising epitopes on TNF. Furthermore, in international patent application WO 92/11383, recombinant antibodies, including CDR-grafted antibodies, specific for TNFα are disclosed. U.S. Pat No. 5,919,452 discloses anti-TNFα chimeric antibodies and their use in treating pathologies associated with the presence of TNFα. Further anti-TNFα antibodies are disclosed in Stephens et al. (Immunology, 85, 668-674, 1995), GB-A-2 246 570, GB-A-2 297 145, US 8,673,310, US 2014/0193400, EP 2 390 267 B1, US 8,293,235, US 8,697,074, WO 2009/155723 A2 and WO 2006/131013 A2.

Currently approved anti-TNFα antibodies include (i) infliximab, a chimeric IgG anti-human monoclonal antibody (Remicade®); (ii) etanercept, a TNFR2 dimeric fusion protein, with an IgG1 Fc (Enbrel®); (iii) adalimumab, a fully human monoclonal antibody (mAb) (Humira®), (iv) certolizumab, a PEGylated Fab fragment (Cimzia®) and (v) golimumab, a human IgGIK monoclonal antibody (Simponi®). Moreover, various biosimilars are in development. Therefore, in one embodiment of the present invention, the antibody or functional fragment thereof is selected from infliximab, adalimumab, etanercept, certolizumab pegol and golimumab or functional fragments thereof. In another embodiment of the present invention, the at least one antibody or functional fragment thereof is selected from anti-TNFα antibody or functional fragment thereof as disclosed in PCT applications PCT/EP2017/056218, PCT/EP2017/056246, PCT/EP2017/056237 and PCT/EP2017/056227, as originally filed. In yet another embodiment of the present invention, the at least one antibody or functional fragment thereof is an anti-TNFα antibody or functional fragment thereof with a light chain variable domain and/or a heavy chain variable domain comprising complementarity-determining regions (CDRs) with amino acid sequences as disclosed in PCT applications PCT/EP2017/056218, PCT/EP2017/056246, PCT/EP2017/056237 and PCT/EP2017/056227, as originally filed.

In a preferred embodiment of the present invention, the at least one antibody or functional fragment thereof is selected from anti-TNFα antibodies or functional fragments thereof with a light chain variable domain and/or a heavy chain variable domain comprising one or more CDRs with amino acid sequences as disclosed in SEQ ID NO:7, 9, 12, 14, 24 and 25 of PCT/EP2017/056218, in SEQ ID NO:7-11 and 6 of PCT/EP2017/056246, in SEQ ID NO:7-12 of PCT/EP2017/056237, in SEQ ID NO:1-4, 7 and 6 of PCT/EP2017/056227, and combinations thereof. In another preferred embodiment of the present invention, the at least one antibody or functional fragment thereof is selected from anti-TNFα antibodies or functional fragments thereof with a light chain variable domain and a heavy chain variable domain comprising CDRs with amino acid sequences as disclosed in claim 2 of PCT/EP2017/056218, in claim 2 of PCT/EP2017/056246, in claim 2 of PCT/EP2017/056237 and/or in claim 2 PCT/EP2017/056227, as originally filed. In yet another preferred embodiment of the present invention, the at least one anti-TNFα antibody or functional fragment thereof is selected from the group consisting of anti-TNFα antibodies or functional fragments thereof comprising a heavy chain variable domain amino acid sequence and/or a light chain variable domain amino acid sequence according to claim 4 of PCT/EP2017/056218, claims 5 and 6 of PCT/EP2017/056246, claims 5 and 6 of PCT/EP2017/056237, claim 4 of PCT/EP2017/056227, and combinations thereof.

The particle comprises a buffer. The nature of the buffer is not particularly limited and includes for example all buffers (buffer salts) that ensure stability and activity of the at least one antibody and functional fragments thereof in solution. Preferably, the buffer salts in the particle are suitable to stabilize the pH of a liquid, preferably the feed liquid of step a), at a pH compatible with the at least one antibody or functional fragment thereof, thereby. Compatible in this context is to be understood to mean being conducive to stability and activity of the at least one antibody or functional fragment thereof. For example the buffer or buffer salt may stabilize the pH in the feed liquid at a physiological pH. In one embodiment of the present invention, the buffer is one or more buffers (buffer salts) selected from the group consisting of acetate buffer; citrate buffer; histidine buffer; succinate buffer; phosphate buffer; hydroxymethylaminomethane (TRIS) buffer; and combinations thereof.

The particle prepared by the present invention comprises at least one sustained release polymer. The sustained release polymer used in the inventive method for the preparation of the particle is not particularly limited. Examples of sustained release polymers suitable for the present invention include poly(ethyl acrylate, methyl methacrylate, trimethylammonioethyl methacrylate chloride) 1 : 2 : 0.1 (e.g. Eudragit® RS 30D); poly(ethyl acrylate, methyl methacrylate, trimethylammonioethyl methacrylate chloride) 1 : 2 : 0.2 (e.g. Eudragit® RL 30D); poly(ethylacrylate, methylmethacrylate) 2:1 (e.g. Eudragit® NE 30D, Eudragit® NE 40D, Eudragit® NM 30D); ethylcellulose aqueous dispersions (such as Aquacoat® ECD and Surelease®); polyvinyl acetate; polyvinyl acetate aqueous dispersion stabilized with povidone and sodium lauryl sulfate (e.g. Kollicoat® SR 30D); aqueous dispersion consisting of about 27% polyvinyl acetate, 2.7% povidone and 0.3% sodium lauryl sulfate (i.e. Kollicoat® SR 30D); hydroxypropyl methylcellulose (HPMC); HPMC 2208 type with a viscosity at 2 wt.-% in water at 20°C between 3 and 1,000 mPa·s, preferably HPMC 2208 type with a viscosity at 2 wt.-% in water at 20°C between 78 and 117 mPa·s (e.g. Methocel K100); and combinations thereof. In one embodiment of the present invention, the sustained release polymer is not biodegradable.

In another embodiment of the present invention the at least one sustained release polymer is selected from group consisting of poly(ethyl acrylate, methyl methacrylate, trimethylammonioethyl methacrylate chloride) 1 : 2 : 0.1 (e.g. Eudragit® RS 30D); poly(ethyl acrylate, methyl methacrylate, trimethylammonioethyl methacrylate chloride) 1 : 2 : 0.2 (e.g. Eudragit® RL 30D); poly(ethylacrylate, methylmethacrylate) 2:1 (e.g. Eudragit® NE 30D, Eudragit® NE 40D, Eudragit® NM 30D); ethylcellulose aqueous dispersions (such as Aquacoat® ECD and Surelease®); polyvinyl acetate; polyvinyl acetate aqueous dispersion stabilized with povidone and sodium lauryl sulfate (e.g. Kollicoat® SR 30D); aqueous dispersion consisting of about 27% polyvinyl acetate, 2.7% povidone and 0.3% sodium lauryl sulfate (i.e. Kollicoat® SR 30D); hydroxypropyl methylcellulose (HPMC); HPMC 2208 type with a viscosity at 2 wt.-% in water at 20°C between 3 and 1,000 mPa·s, preferably HPMC 2208 type with a viscosity at 2 wt.-% in water at 20°C between 78 and 117 mPa·s (e.g. Methocel K100); and combinations thereof. In a preferred embodiment of the present invention the at least one sustained release polymer is selected from group consisting of poly(ethyl acrylate, methyl methacrylate, trimethylammonioethyl methacrylate chloride) 1 : 2 : 0.1; HPMC 2208 type with a viscosity at 2 wt.-% in water at 20°C between 78 and 117 mPa·s; and combinations thereof; more preferably poly(ethyl acrylate, methyl methacrylate, trimethylammonioethyl methacrylate chloride) 1 : 2 : 0.1.

The sustained release polymer may be provided in any form that allows dispersing the polymer in the feed liquid. In a preferred embodiment of the present invention, the polymer is provided as an aqueous dispersion (suspension), e.g. a ready-to-use aqueous dispersion, which can be directly added to the feed liquid. In the particle, at least one sustained release polymer may be used. However more than one, e.g. two, three or four, sustained release polymers can be used as polymers of the particle.

The at least one sustained release polymer is not particularly limited as long as it does not affect stability, activity and solubility of the at least one antibody or functional fragment thereof, and as long as it allows the feed liquid to be processed by spray drying, when dispersed therein. Preferably, the at least one sustained release polymer ensures a sustained release of the at least one antibody or functional fragment thereof from particle. The term "sustained release" is known in the art. As used herein, the term "sustained release" may be used to describe a release of the active agent from a particle or multiparticulate solid dosage form, such that a substantial fraction of antibody or functional fragment thereof is released from the particle or multiparticulate solid dosage form in a continuous manner, upon exposure to a aqueous environment (aqueous solution) over a prolonged period of time, e.g. over at least 6 h, at least 10 h, at least 14 h, at least 18 h, or at least 24 h. Means to determine a concentration of an antibody or fragment thereof in an aqueous solution/environment are known in the art and include for example measuring the absorbance at 280nm or using a colorimetric, reagent-based protein assay like the Bradford assay, or measuring by size-exclusion chromatography or ELISA. The term an "aqueous solution" as used herein may refer to a solution or suspension of which a large part is water (e.g. more than 30 wt.-%, preferably more than 40 wt.-%, preferably more than 50 wt.-%, preferably more than 60 wt.-%, most preferably more than 70 wt.-% of water). This includes intestinal fluid. The aqueous solution preferably comprises buffers (aqueous buffer solution). It is to be understood that throughout the present disclosure, whenever referring to dissolution of, or recovery of antibodies or functional fragments thereof from, a particle/multiparticulate solid dosage form (as in the section just below), e.g. by continuously immersing a particle/multiparticulate solid dosage form in an aqueous (buffer) solution under continuous (constant) agitation, for example the following standard testing setup, or a related standard testing setup known to the person skilled in the art, can be used: The release of the at least one antibody or functional fragment thereof can be evaluated using a standard dissolution apparatus I (baskets), II (paddle), III (reciprocating cylinder) or apparatus IV (flow through cell), where the buffer (i.e. aqueous buffer solution) is equilibrated at 37°C. The buffer volume used for dissolution testing can be adapted for instance using mini-vessels in the apparatus I or II to allow reduction of volume required and to be more bio-relevant. Release of antibodies or functional fragments thereof during dissolution can be quantified offline by an ELISA method.

In one embodiment of the present invention, the at least one sustained release polymer in the particle, allows the recovery of at least 40 %, preferably at least 50 %, more preferably at least 60 %, even more preferably at least 70 %, even more preferably at least 80 %, even more preferably at least 85%, even more preferably at least 90%, even more preferably at least 93%, most preferably at least 95 %, of the at least one antibody or functional fragment thereof from the particle (or the multiparticulate solid dosage form) within a defined time period (e.g. 4 h, or 6 h, or 8 h, or 10 h, or 12 h, or 14 h, or 16 h, or 18 h, or 20 h, or 22 h, or 24 h, or 26 h, or 28 h, or 30 h, etc.) of continuously immersing the particles (or the multiparticulate solid dosage form) in an aqueous solution, e.g. at a temperature of between 25 °C and 45 °C (preferably 25-40 °C, more preferably about 37 °C) and under continuous agitation. In another embodiment of the present invention, the at least one sustained release polymer in the particle, allows a sustained release of the at least one antibody or functional fragment thereof over a time period of at least 5 h, preferably at least 10 h, more preferably at least 15 h, even more preferably at least 20 h, most preferably at least 24 h, etc., upon continuously immersing the particle (or the multiparticulate solid dosage form) in an aqueous solution or suspension. In an alternative embodiment of the present invention, the at least one sustained release polymer in the particles prepared by the present invention, allows a sustained release of the at least one antibody or functional fragment thereof over a time period of at least 8 h, at least 10 h, at least 12 h, at least 14 h, or at least 16 h upon continuously immersing the particles (or multiparticulate solid dosage form) in an aqueous solution. In another alternative embodiment the at least one sustained release polymer in the particles, gives rise to a release profile for the at least one antibody or functional fragment thereof, where a sustained release with a substantially constant release rate of at least 80 %, preferably at least 90 %, of the at least one antibody or fragment thereof in the particles is achieved over 4-32 h (e.g. over 4 h, or 6 h, or 8 h, or 10 h, or 12 h, or 14 h, or 16 h, or 18 h, or 20 h, or 22 h, or 24 h, or 26 h, or 28 h, or 30 h, etc.), upon continuously immersing the particles (or the multiparticulate solid dosage form) in an aqueous solution.

In particular for conditions that affect a section of the gastrointestinal tract (gastrointestinal diseases), including the ileum and large intestine, such as Crohn's Disease and ulcerative colitis, a sustained release particle/solid dosage form of an active biological agent in the form of an antibody or functional fragment thereof, exhibiting limited systemic absorption can be desirable.

The inventive method, in its most general form, comprises as first step, step a), preparing a feed liquid comprising the at least one antibody or functional fragment thereof, the buffer and the at least one sustained release polymer, all in an aqueous suspension, and optionally a surfactant and/or a plasticizer and/or a filler. The individual components of the feed liquid, i.e. at least one antibody or functional fragment thereof, the buffer and the at least one sustained release polymer, as defined above, may be provided in any form that allows bringing them into the feed liquid. For example the individual component may be provided as powders, granules or suspended or dissolved in a solvent depending on the component, and then depending on the component can be dissolved or dispersed in the aqueous suspension, or may be provided already dissolved or dispersed in a liquid.

The term "aqueous suspension" as used herein refers to a suspension, wherein at least 60 %, preferably at least 70 %, more preferably at least 80 %, even more preferably at least 90 %, even more preferably at least 95 %, 97 %, 98 %, 99 %, or 99.5 %, even more preferably at least 99.9 %, most preferably 100 %, of the solvent used is water. In one embodiment of the present invention, the feed liquid is an aqueous suspension wherein 100 % of the solvent used is water.

The total solid content of the feed liquid is not particularly limited as long as it allows processing of the feed liquid by spray drying, and as long as stability, activity, and minimal irreversible interactions with other components of the final particle, are ensured. According to one embodiment of the present invention, the total solid content in the feed liquid (w/w) is between 0.1 and 30 wt.-%, preferably between 0.1 and 15 wt.-%, more preferably between 0.5 and 10 wt.-%, even more preferably between 1 and 10 wt.-%, even more preferably between 5 and 10 wt.-%.

The manner in which the at least one antibody or functional fragment thereof is introduced into the feed liquid is not particularly limited. For example, the at least one antibody or functional fragment thereof can be added as a powder containing antibodies or functional fragments thereof, and thereby directly reconstitute the antibody or functional fragment thereof in the feed liquid. The term "powder" as used in this context is to be understood in its broadest meaning, including fine particles as well as larger particles and granules. Alternatively, the at least one antibody or functional fragment thereof can be added already in solution or suspension, e.g. as part of a buffered aqueous solution. According to one embodiment, the latter is preferred. For example the at least one antibody or functional fragment thereof may be in solution in a buffer system, which is mixed with an aqueous dispersion of the at least one sustained release polymer. The total solid content in the feed liquid can then be controlled by pre-dilution of the antibody solution and/or the aqueous polymer dispersion or by dilution of the final feed liquid. Moreover, preferably the at least one antibody or functional fragment thereof is dissolved in the feed liquid of step a).

The concentration of the at least one antibody or functional fragment thereof in the feed liquid is not particularly limited as long as the concentration allows processing of the feed liquid by spray drying, and as long as stability, activity and minimal irreversible interactions with other components of the feed liquid are ensured for the at least one antibody or functional fragment thereof. In one embodiment of the present invention the feed liquid comprises 0.001 to 35 wt.-%, 0.01 to 30 wt.-%, preferably 0.1 to 15 wt.-%, more preferably 0.5 to 10 wt.-%, even more preferably 1 to 5 wt.-%, even more preferably about 3.5 to 4.5 wt.-%, of the at least one antibody or functional fragment thereof, relative to the total solid content. In an alternative embodiment of the present invention the feed liquid comprises 0.001 to 35 wt.-%, preferably 0.1 to 30 wt.-%, more preferably 1 to 25 wt.-%, even more preferably 5 to 25 wt.-%, even more preferably 10 to 25 wt.-%, even more preferably 12 to 23 wt.-%, of the at least one antibody or functional fragment thereof, relative to the total solid content. In another embodiment of the present invention the feed liquid comprises 0.01-100 mg/ml, preferably 0.1-50 mg/ml, more preferably 0.5-30 mg/ml, even more preferably 1-25 mg/ml, even more preferably 5-25 mg/ml, even more preferably 5-15 mg/ml, of the at least one antibody or functional fragment thereof.

In an alternative embodiment of the present invention, the amount of the antibody in the feed liquid, relative to the total solid content is such as to result in an amount of antibody or functional fragment thereof in the particles prepared by the method of the present invention that allows the administration of a therapeutically effective dose of the at least one antibody or functional fragment thereof as a single unit dose, for example in the form of a tablet, spheroid, sachet/stick pack or capsule comprising multiple particles. The term "administration" relates to the manner and form in which the composition comes into first contact with the body of a patient. The particles prepared by the inventive method may be intended for oral administration or administration in any other way that results in the accumulation of the particles at the intended site of local application and/or absorption into body tissue. Preferably the particles of the present invention are intended for oral administration. A "therapeutically effective dose" is the amount of the at least one antibody or functional fragment thereof required to provide the desired therapeutic effect. The exact amount may vary for different antibodies or functional fragments thereof and/or for individual patients, but can be determined by one skilled in the art.

The feed liquid comprises a buffer. The nature of the buffer is not particularly limited and preferably includes all buffers that are conducive to stability and activity of the at least one antibodies or functional fragment thereof in solution. In one embodiment of the present invention, the buffer is preferably at least one (but also more than one) buffer or buffer salt that stabilizes the pH of the feed liquid at a pH compatible with the at least one antibody or functional fragment thereof. In an alternative embodiment, the buffer is preferably at least one (but also more than one) buffer or buffer salt that stabilizes the pH of the feed liquid at a physiological pH. In another embodiment, the buffer is selected from the group consisting of acetate buffer; citrate buffer; histidine buffer; succinate buffer; phosphate buffer; hydroxymethylaminomethane (TRIS) buffer; and combinations thereof. The buffer may be present in the feed liquid in any amount that ensures stability and activity of antibodies and functional fragments thereof in solution. In one embodiment of the present invention, the feed liquid comprises 0.01-20 wt.-%, preferably 0.1-15 wt.-%, e.g. about 1-5 wt.-%, about 1.5 to 4 wt.-%, about 2-3 wt.-%, about 2-5 wt.-%, about 2.3 wt.-%, about 4.5 wt.-%, or about 15 wt.-%, buffer, relative to weight of the feed liquid

The sustained release polymer as defined above may be used in any form that allows dispersion in the feed liquid. In one embodiment of the present invention, the sustained release polymer is already in solution or suspension, preferably as part of an aqueous suspension (aqueous dispersion), and is added as such to the feed liquid. In an alternative embodiment of the present invention, the at least one sustained release polymer is added to the feed liquid as solids e.g. in the form of a powder or granules. According to a preferred embodiment, the at least one sustained release polymer is added to the feed liquid in the form of an aqueous suspension (aqueous dispersion). According to one embodiment where the at least one sustained release polymer is added to the feed liquid in the form of an aqueous suspension, the aqueous suspension comprising the at least one sustained release polymer may be diluted to a concentration at which it can be added to the feed liquid without requiring further dilution of the feed liquid after that.

The amount of sustained release polymer in the feed liquid is not particularly limited as long as the feed liquid can be used for processing by spray drying, and at the same time for the at least one antibody or functional fragment thereof stability, activity and minimal irreversible interaction with other components of the final particle are ensured. In one embodiment of the present invention, the feed liquid comprises a concentration of sustained release polymer that maximizes processing speed (i.e. spray rate and drying time), while minimizing clogging of the tubing and the spray nozzle. In another embodiment of the present invention, the feed liquid comprises 5 to 95 wt.-%, preferably 75 to 90 wt.-%, more preferably 83 to 85 wt.-%, alternatively preferably e.g. 5 to 70 wt.-%, 5 to 50 wt.-%, 6 to 49 wt.-%, 15 to 45 wt.-%, 15 to 40 wt.-%, 25 to 35 wt.-%, 20 to 30 wt.-%, 30 to 40 wt.-%, 45 to 55 wt.-%, 50 to 60 wt.-%, 55 to 75 wt.-%, 60 to 70 wt.-%, 75 to 85 wt.-%, 80 to 70 wt.-%, or 85 to 95 wt.-%, , of the at least one sustained release polymer, relative to the total solid content.

The mass ratio of different components in the feed liquid to each other, in particular the mass ratio of the sustained release polymer relative to the at least one antibody or functional fragment thereof, can be adjusted to improve processability of the feed liquid as well as the properties of the resulting particles. Moreover by adjusting the ratio of the at least one sustained release polymer to the at least one antibody or functional fragment thereof feed liquid, the release rate of the at least one antibody or functional fragment thereof can be modified, such that a higher ratio results in a slower release rate. Thereby the release rate of the at least one antibody or functional fragment thereof from the particles can be adapted to individual requirements of the antibody or functional fragment thereof used, the site of release and the condition to be treated. According to one embodiment of the present invention, the mass ratio of the at least one sustained release polymer (S) to the at least one antibody or functional fragment thereof (A) modifies the release rate of the at least one antibody or functional fragment thereof, such that a higher ratio results in a slower release rate. According to another embodiment of the present invention, the feed liquid comprises a mass ratio of the at least one sustained release polymer to the at least one antibody or functional fragment thereof (S/A) of between 0.1 and 500, preferably 0.1 and 200, more preferably 0.5 and 100, even more preferably 5 and 90, even more preferably 10 and 85, even more preferably 15 and 75, even more preferably 15 and 60, even more preferably 15 and 50, even more preferably 20 and 50, even more preferably 21 and 30.

The feed liquid optionally comprises at least one further excipient. The term "excipient," as used herein, refers to a non-therapeutic agent added to the feed liquid to provide a desired consistency, viscosity or stabilizing effect. Further excipients include for example filler, plasticizer, pore forming agent, viscosity-reducing agent, surfactants , divalent cations, antioxidants, humectants, protective colloids, dyes, fillers and combinations thereof. The optional further excipients, in the amounts used, are compatible with other components of the feed liquid. Preferably, the optional further excipients are conducive to stability, activity and/or recovery from the final particles, of the at least one antibody or functional fragment thereof.

According to one embodiment of the present invention, the feed liquid in step a) comprises at least one filler. A filler may improve the properties of the resulting particles, and may increase long-term stability of the at least one antibody or functional fragment thereof in the particle. The filler to be used in the feed liquid is not particularly limited. In one embodiment of the present invention, the filler is selected from the group consisting of dextrose, lactose, lactose monohydrate, lactose anhydrous, mannitol, sorbitol, xylitol, sucrose, glucose, raffinose, trehalose, dicalcium phosphate, oxides of magnesium or silicon, titanium carbonate, calcium carbonate, magnesium phosphate, porous calcium phosphate or porous magnesium phosphate, amino acids such as arginine, histidine, glycine, alanine, lysine, proline, leucine, glutamic acid, serine, aspartic acid and asparagine, and respective salts thereof, propylene glycol, polyethylene glycol, and combinations thereof; preferably dextrose, sucrose, mannitol, sorbitol, xylitol, trehalose, amino acids such as arginine, histidine, glycine, alanine, lysine, proline, leucine, glutamic acid, serine, aspartic acid and asparagine, and respective salts thereof, dicalcium phosphate, and combinations thereof; more preferably sorbitol, trehalose, sucrose, mannitol, or amino acids such as arginine, histidine, glycine, alanine, lysine, proline, leucine, glutamic acid, serine, aspartic acid and asparagine, and respective salts thereof, even more preferably trehalose, mannitol, sucrose, and combinations thereof, even more preferably trehalose or mannitol. The amount of filler that may be used in the feed liquid is not particularly limited. In one embodiment of the present invention, the feed liquid comprises 0.01 to 40 wt.-%, preferably 0.5 to 30 wt.-%, more preferably 2 to 20 wt.-%, of the at least one filler relative to the total amount of solids in the feed liquid.

The feed liquid in step a) optionally further comprises at least one plasticizer. The plasticizer to be used in the feed liquid is not particularly limited. In one embodiment of the present invention, the plasticizer is selected from the group consisting of triethyl citrate (TEC), dibutyl sebacate (DBS), polyethylene glycol, acetyl triethyl citrate, butyl citrate, polysorbates, 1, 2-propylene glycol, and combinations thereof. In a preferred embodiment of the present invention, the plasticizer is selected from the group consisting of TEC and DBS, and combinations thereof. The amount of plasticizer that may be used in the feed liquid is not particularly limited. In one embodiment of the present invention, the feed liquid comprises 0.1-30 wt.-%, preferably 0.5-20 wt.-%, more preferably about 0.5-5 wt.-%, plasticizer, relative to the total amount of polymer solids in the feed liquid.

The feed liquid in step a) optionally further comprises a pore forming agent. The pore forming agent to be used in the feed liquid is not particularly limited. In one embodiment of the present invention, the pore forming agent is hypromellose or polyvinyl alcohol-polyethylene glycol (PVA-PEG) graft copolymer. Moreover, the feed liquid in step a) optionally further comprises a viscosity-reducing agent. A viscosity-reducing agent can improve processability of the feed liquid and can be used to control droplet formation during spraying. The viscosity-reducing agent preferably is selected from the group consisting of arginine succinate, arginine glutamate or arginine hydrochloride, and combinations thereof.

According to another embodiment of the present invention, the feed liquid in step a) further comprises surfactant. In one embodiment of the present invention, the surfactant is selected from the group consisting of polysorbates such as polysorbates 20, 28, 40, 60, 65, 80, 81 and 85; poloxamers such as poloxamers 124, 181, 188, 237, 331, 338 and 407, glyceryl monostearate, polyethoxylated castor oil, PEG-40 hydrogenated castor oil, macrogol 15 hydroxystearate, polyoxyl 15 hydroxystearate, caprylocaproyl macrogol-8 glyceride, D-α-tocopherol polyethylene glycol 1000 succinate, glyceryl monostearate, lecithin, sorbitan monopalmitate, cetyl alcohol, oleyl alcohol, sodium glycolate, sodium de(s)oxycholate, block copolymer of polyethylene and polypropylene glycol, polyethylene glycol, polypropylene glycol, alkyl poly(ethylene oxide), alkyl glycoside, alkyl polyglucoside, octyl glucoside, decyl maltoside, and combinations thereof. The surfactant that may be used in the feed liquid is not particularly limited. In one embodiment of the present invention, the feed liquid comprises 0.01 to 2.0 wt.-%, preferably 0.05 to 1 wt.-%, more preferably about 0.1-0.5 wt.-% of the sufactant, relative to the total amount of solids in the feed liquid.

Other further excipients that are optionally comprised in the feed liquid are divalent cations (e.g., Ca2+, Zn2+, Mg2+, and Mn2+), antioxidants, humectants, protective colloids, dyes, fillers. In yet another embodiment of the present invention, the feed liquid comprises at least one further excipient selected from the group consisting of divalent cations (e.g., Ca2+, Zn2+, Mg2+, and Mn2+), antioxidants, humectants, protective colloids, dyes, and combinations thereof.

The viscosity of the feed liquid preferably is such as to allow processing of the feed liquid by spray drying. The viscosity of the feed liquid is dependent on its composition, i.e. components and their concentration in the feed liquid.

The individual components of step a) can be blended to give rise to the feed liquid for example by any conventional mixing device. Such mixing devices are known in the art and include for example a paddle mixer or a magnetic stirring mixer. The mixing device may be an integral part of the spray drier used for step b).

In step b) of the inventive method, the feed liquid is sprayed through a nozzle into a primary drying chamber to form droplets, wherein the droplets are dispersed in a drying gas. For step b), as well as step c) and d), of the inventive method spray drying equipment is used. Spray drying equipment (spray drier) suitable for use with a feed liquid comprising an active agent is known in the art. Suitable spray drier for use with the inventive method include for example those available from Büchi Labortechnik, ProCepT, SPX, or GEA. In accordance with the present invention it is important that during spray drying specific parameters and conditions, including for example temperature and pressure to which the feed liquid is exposed to, as well as processing time, in order to preserve activity and stability of the at least one antibody or functional fragment thereof in the feed liquid, while ensuring the formation of intact particles.

According to one embodiment where medium-scale spray drying equipment is used, the feed liquid is sprayed through the nozzle at a spray rate of between 0.5 and 100 g/min, preferably 1 and 70 g/min, more preferably 10 and 60 g/min, most preferably 15 and 55 g/min. According to an alternative embodiment where small-scale spray drying equipment is used the feed liquid is sprayed through the nozzle at a spray rate of between 0.1-10 g/min, preferably 0.5-5 g/min, more preferably 1.5-4.5 g/min, even more preferably 2-3 g/min.

The feed liquid is sprayed through the nozzle into a primary drying chamber, equipped or not with extended column(s). A nozzle type suitable for step b) is for example a bi-fluid nozzle or a ultrasonic nozzle. According to one embodiment of the present invention the nozzle in step b) of the inventive method is a bi-fluid nozzle or an ultrasonic nozzle. According to a preferred embodiment the nozzle is a bi-fluid nozzle. According to an embodiment where the nozzle is a bi-fluid nozzle, the nozzle gas pressure is for example lower than 200 kPa, preferably lower than 100 kPa and/or the nozzle has a diameter of 0.1-2 mm, preferably 0.2-0.1 mm, more preferably 0.4 to 0.8 mm, most preferably about 0.6 mm According to an alternative embodiment where the nozzle is an ultrasonic nozzle, the ultrasonic nozzle is for example operated at a frequency of 20 to 120 kHz, preferably 40 to 80 kHz, more preferably about 60 kHz

The formed droplets are dispersed in a drying gas. The drying gas is not particularly limited as long as it is not detrimental to stability or activity of the at least one antibody or functional fragment thereof in the feed liquid. A suitable drying gas is for example be air. The drying air enters the primary drying chamber through an inlet and exits the primary drying chamber through an outlet. According to one embodiment, the inlet air flow of the drying gas is between 0.2 and 5 m³/min, preferably 0.2 and 3 m³/min, more preferably 0.3 and 2.5 m³/min. The inlet temperature of the drying gas, according to one embodiment, is between 50 °C and 200 °C, preferably between 80°C and 190°C, more preferably between 90 °C and 170 °C, even more preferably between 90 °C and 150 °C, even more preferably between 100 °C and 140 °C. According to a further embodiment, the outlet temperature of the drying gas, with the particles dispersed therein, is between 25 °C and 120 °C, preferably between 40°C and 100°C, more preferably between 45 °C and 90 °C, even more preferably between 45 °C and 80 °C, even more preferably between 50 °C and 75 °C.

It is to be understood that the above numerical ranges for parameters column height, spray rate, nozzle size, nozzle air, and inlet air flow of the drying gas are optimized for small- to medium-scale spray drying equipment. These parameters require further optimization for use with large-scale spray drying equipment. How to adjust these parameters to large-scale spray drying equipment on the basis of the present application is known to the person skilled in the art.

According to step c) of the method of the present invention, the droplets are dried by evaporating at least a portion of the liquid in the droplets into the drying gas, to form particles dispersed in the drying gas. According to one embodiment, the droplets dispersed in the drying air are dried until dried particles are obtained. The term "dried" or "dry" when referring to a particle (e.g. as in "dry particle") may refer to a particle containing less than 20 %, preferably less than 10 %, more preferably less than 7 %, even more preferably less than 5 %, even more preferably less than 3 %, even more preferably less than 2% and most preferably less than 1.5 % residual moisture (i.e the residual solvent content). Preferably the after step d) the residual moisture in the particles is less than 15 wt.-%, preferably less than 10 wt.-%, e.g. less than 9 wt.-%, 8 wt.-%, 7 wt.-%, 6 wt.-%, 5 wt.-%, 4 wt.-%, 3 wt.-%, 2 wt.-%, or 1 wt.-%" relative to the total weight of the particle after step d). One method for determining the residual moisture of particles or multiparticualte solid dosage forms is the Loss on Drying (LOD) technique. For example the amount of moisture contained in particles (or in a multiparticulate solid dosage form) can be gravimetrically measured using LOD at 105 °C for 1 hour.

According to step d) of the method of the present invention, the drying gas with the particles dispersed therein are directed through a particle concentration means to recover the particles. The particle concentration means may be part of the primary drying chamber or may be a separate chamber. According to one embodiment, the particles dispersed in the drying gas exit the drying chamber at the outlet and are directed through the particle concentration means to recover the particles. According to another embodiment, the formed particles may further dry while being directed through the particle concentration means. According to a further embodiment of the present invention, the particle concentration means separates the particles from the drying gas by removing between 50 vol.-% to 100 vol.-% of the drying gas.

Concentration means for recovering spray-dried particles dispersed in a drying gas are known in the art. According to one embodiment of the present invention, the particle concentration means in step d) comprises a cyclone separator. According to a further embodiment the temperature of the drying gas with the particles dispersed therein before the cyclone is between 10 °C and 70 °C, preferably 15°C and 60°C, more preferably 20 °C and 55 °C, even more preferably 25 °C and 50 °C.

The activity and stability of antibodies and functional fragments thereof as used for the present invention are very sensitive to external stress like temperature fluctuations and particularly to elevated temperatures. Therefore, in accordance with the method of the present invention the temperature of the at least one antibody and functional fragment thereof during drying preferably is such as to protect the activity and stability of the antibodies and functional fragments thereof and at the same time allow efficient drying of the particles.

The particles prepared by the inventive method preferably are generally spherical or spheroidal in shape and have an equivalent spherical diameter of less than 500 µm, preferably less than 250 µm more preferably less than 100 µm. How to determine the equivalent spherical diameter of particles is known in the art. According to alternative embodiment of the present invention the particles have a sphericity of more than 0.6, preferably more than 0.7, more preferably more than 0.8, even more preferably more than 0.9.

According to one embodiment of the present invention, the particles after step d) have a particle-size distribution (PSD) D50 of 0.5-200 µm, preferably 1-100 µm, more preferably 5-75 µm, even more preferably 5-50 µm, even more preferably 10-40 µm, even more preferably 10-30 µm. According to another embodiment of the present invention, the particles after step d) have a particle-size distribution D90 such that at least 90 % of the particles have a particle size of 0.5-200 µm, preferably 1-120 µm, more preferably 5-100 µm, even more preferably 10-90 µm, even more preferably 20-80 µm, even more preferably 30-70 µm. The term "particle-size distribution" or "PSD" as used herein refers to the relative amount (w/w) of particles present according to size. The PSD D50 is the diameter of the particle that 50% of a sample's mass is smaller than and 50% of a sample's mass is larger than, i.e. the median particle diameter. The PSD D50 can be determined for example using sedimentation techniques, photoanalysis, microscopy or laser diffraction analysis. According to the present invention the PSD D50 is preferably determined by laser diffraction. According to one embodiment of the present invention each particle prepared by the inventive method has a predetermined axis and the same predetermined cross-sectional profile, such that at least 50 %, at least 80 % of the particles, preferably 90 %, more preferably 95 %, by number, have a median aspect ratio between 0.6 and 1.8, the aspect ratio being defined as particle length along the predetermined axis divided by the smallest cross-sectional dimension. According to a further embodiment, the median aspect ratio is above 0.7 preferably above 0.8, more preferably above 0.9, and below 1.7, preferably 1.6, more preferably below 1.5, more preferably 1.4, even more preferably below 1.3, even more preferably below 1.2, most preferably about 1. According to yet another embodiment the particles have a span of aspect ratio of less than 0.9, preferably less than 0.8, more preferably less than 0.7, even more preferably less than 0.6, most preferably less than 0.5. For further details regarding aspect ratio, predetermined axis, predetermined cross-sectional profile and span (including definitions and embodiments), it is referred to disclosure of EP 2 512 453.

The amount of the at least one antibody or fragment thereof in the particles after step d) is not particularly limited, and depends on the amount of the other components and the residual moisture in each particle. According to one embodiment of the present invention, the particles comprise 0.01 to 50 wt.-%, preferably 0.1 to 30 wt.-%, more preferably 0.5 to 25 wt.-%, even more preferably 0.5 to 20 wt.-%, even more preferably 0.5 to 10 wt.-%, even more preferably 1 to 7 wt.-% of the at least one antibody or functional fragment thereof, relative to the total weight of the particles after step d). The particles prepared by the inventive method may be intended for oral or rectal. According to preferred embodiment the particles are intended for oral administration.

In one embodiment of the present invention the antibody or functional fragment thereof is suitable for use in the topical treatment in the gastrointestinal tract of a patient. The term "topical treatment" in the context of the present invention, is used to describe the local application a solid dosage form (e.g. comprising multiple particles), as opposed to the systemic application of a dosage form comprising antibodies or functional fragments thereof, e.g. by infusion, injection or implantation. The term "gastrointestinal tract" as used herein describes the system of organs of the human body, that includes all structures between mouth and anus, forming a continuous passage, and is responsible for digesting ingested material, absorbing nutrients and expelling faeces. The term "patient" as used herein refers to a living organism suffering from or prone to a condition that can be treated or prevented by the administration of the at least one antibody or functional fragment thereof. In a preferred embodiment, the patient is a human.

A pharmaceutical composition in the form of multiple particles (i.e. multiparticulate solid dosage form) allows once-daily, several times a day, once-in-two days, etc., delivery of the above classes of antibodies and functional fragments thereof. The topical treatment in the gastrointestinal tract, e.g. the ileum or the large intestine, ensures specific targeting of the gastrointestinal wall, for enhanced treatment of diseases of the ileum and large intestine, by providing high local concentration of antibody or functional fragment thereof, while minimizing side effects that occur because of release of drugs in the upper gastrointestinal tract or unnecessary systemic absorption.

Therefore in another embodiment of the present invention the particles prepared by the inventive method are suitable for use in the treatment of a disease in the gastrointestinal tract (gastrointestinal diseases), preferably in the ileum and the large intestine. Such diseases include e.g. IBD, cancer (such as colorectal cancer or small intestine cancer), celiac disease, infections (such as Clostridium difficile infection) of the small intestine and the colon, and diarrhea. In a preferred embodiment of the present invention the particles prepared by the inventive method are for use in the treatment of IBD, e.g. Crohn's disease or ulcerative colitis.

In one embodiment of the present invention, the particles prepared by the method of the present invention are for oral administration. "Oral administration" in context of the present invention means the introduction of the particles for example comprised in a multiparticulate solid dosage form into gastrointestinal tract via the mouth.

According to a further aspect of the present invention, in a further step, a multiparticulate solid dosage form, e.g. a sachet/stick pack, straw device (Xstraw®), a tablet, a mini tablet, a pellet, a bead, a sphere (or spheroid), a mini sphere a or a capsule (e.g. a hard or soft gelatin capsule or HPMC capsule) is provided, comprising multiple particles prepared by the inventive method according to one of the embodiments described above. How to prepare sachets/stick packs, straw devices, tablets, spheroids, spheres, pellets, beads or capsules comprising multiple particles is known in the art. For example, a multiparticulate solid dosage form, e.g. a tablet, mini tablet, pellet, sphere, mini sphere, spheroid, and the like, may be prepared by direct compression of a mixture of a plurality of particles, a compression binder and optionally one or more excipient. Alternatively, a multiparticulate solid dosage form, e.g. a tablet, mini tablet, pellet, sphere, mini sphere, spheroid, and the like, may be prepared by extrusion of an extrusion mass comprising a plurality of particles, a binder and optionally one or more excipient, to form an extrudate, and then forming individual units (each comprising multiple particles) e.g. by spheronization or by cutting the extrudate into equally sized units.

The multiparticulate dosage form (sachet/stick pack, straw device, spheroid, tablet, capsule, etc.) may for example comprise a total amount of the at least one antibody or functional fragment thereof suitable for oral administration to a human patient. In another embodiment, the multiparticulate dosage form (sachet/stick pack, straw device, tablet, spheroid, capsule, etc.) comprises a therapeutically effective dose of the at least one antibody or functional fragment thereof suitable for oral administration to a human patient. According to a further embodiment, the multiparticulate solid dosage form is intended for oral administration.

According to one embodiment of the present invention at least one additional coating in the form of a delayed release coating is applied to the multiparticulate solid dosage form. A delayed release coating within the meaning of the present invention is a coating that prevents the release of the antibody or functional fragment thereof from the solid dosage form, until a specific event, e.g. in the form of a chemical or enzymatic trigger or the lapse of a defined amount of time immersed in solution, occurs.

In a preferred embodiment, the multiparticulate solid dosage form prepared from a plurality of particles prepared by the method of the present invention is for oral administration, with a delayed release coating that prevents the release of the at least one antibody or functional fragment thereof before the ileum, preferably before the terminal ileum, more preferably before the ileocolonic region, alternatively before the ascending colon, before the transverse colon or before the descending colon, of the gastrointestinal tract. The ileocolonic region is the region of the gastrointestinal tract where the small intestine merges with the large intestine. The large intestine is the penultimate section of the gastrointestinal tract and can be further subdivided into cecum, colon and rectum. The colon is further subdivided into ascending, transverse and descending colon. The terminal ileum is the penultimate section of the small intestine and is directly adjacent to the cecum.

The approach for applying the delayed release coating is not particularly limited as long as it does not affect the stability and activity of the at least one antibody or functional fragment thereof in the particles. Methods for applying delayed release coatings are known in the art. In one embodiment of the present invention the delayed release coating is applied by spray coating, preferably fluidized-bed spray coating.

Coating materials for the delayed release, in particular for targeted release in the ileum or the large intestine, upon oral administration are known in the art. They can be subdivided into coating materials that disintegrate above a specific pH, coating materials that disintegrate after a specific residence time in the gastrointestinal tract and coating materials that disintegrate due enzymatic triggers specific to the microflora of a specific region of the intestines. Coating materials of these three different categories for targeting to the large intestine have been reviewed for example in Bansal et al. (Polim. Med. 2014, 44, 2,109-118). The uses of such coating materials have also been described for example in WO2007/122374A2, WO0176562A1 WO03068196A1 and GB2367002A. In one embodiment of the present invention the delayed release coating comprises at least one component selected from coating materials that disintegrate pH-dependently, coating materials that disintegrate time-dependently, coating materials that disintegrate due to enzymatic triggers in the intestinal environment (preferably in the intestinal environment of the ileum and the large intestine), and combinations thereof.

Preferred coating materials among coating materials that disintegrate pH-dependently are selected from poly vinyl acetate phthalate, cellulose acetate trimellitate, hydroxypropyl methylcellulose phthalate HP-50, HP-55 or HP-55S, cellulose acetate phthalate, hydroxypropyl methylcellulose acetate succinate (HPMCAS), poly(methacrylic acid, ethyl acrylate) 1:1 (Eudragit® L100-55, Eudragit® L30D-55), poly(methacrylic acid, methyl methacrylate) 1:1 (Eudragit® L-100, Eudragit® L12.5), poly(methacrylic acid, methyl methacrylate) 1:2 (Eudragit® S-100, Eudragit® S12,5, Eudragit® FS30D), and combinations thereof. Preferred coating materials among coating materials that disintegrate time-dependently are selected poly(ethyl acrylate, methyl methacrylate) 2 : 1 (e.g. Eudragit® NM 30D, or Eudragit NE 30D); poly(ethyl acrylate, methyl methacrylate, trimethylammonioethyl methacrylate chloride) 1 : 2 : 0.1 (e.g. Eudragit® RS 30D); ethylcellulose (e.g. Surelease® or Aquacoat ECD); poly(ethyl acrylate, methyl methacrylate, trimethylammonioethyl methacrylate chloride) 1 : 2 : 0.2 (e.g. Eudragit® RL 30D); polyvinyl acetate (eg. Kollicoat® SR 30D); and combinations thereof. Preferred coating materials among coating materials that disintegrate due to enzymatic triggers in the large intestinal environment are selected from chondroitin sulfate, pectin, guar gum, chitosan, inulin, lactulose, raffinose, stachyose, alginate, dextran, xanthan gum, locust bean gum, arabinogalactan, cyclodextrin, pullulan, carrageenan, scleroglucan, chitin, curdulan, levan, amylopectin, starch, amylose, resistant starch, azo compounds being degraded by azo bonds splitting bacteria, and combinations thereof. The delayed release coating optionally comprises one or more further excipients.

In one embodiment of the present invention the coating material for the delayed release coating is selected from the coating materials that disintegrate pH-dependently, the coating materials that disintegrate time-dependently, and the coating materials that disintegrate due to enzymatic triggers in the intestinal environment, listed above, and combinations thereof. In another embodiment of the present invention, the delayed release coating comprises a combination of at least one material that disintegrates pH-dependently and at least one coating material that disintegrates due to enzymatic triggers in the large intestinal environment.

For example, a delayed release coating can be designed to focus the delivery of the antibody or functional fragment thereof entirely in the large intestine, beginning at the cecum, and continuing through the ascending, transverse, and descending colon, and ending in the sigmoid colon. Alternatively, for example, a delayed release coating can be designed to begin the delivery of the antibody or functional fragment thereof in the jejunum and end the release in the transverse colon. The possibilities and combinations are numerous.

In one embodiment of the present invention, the delayed release coating comprises a combination of at least one pH sensitive (enteric) polymer, e.g. poly(methacrylic acid, methyl methacrylate) 1:2, and at least one polysaccharide selected from chondroitin sulfate, cyclodextrin, chitosan, dextran, arabinogalactan, amylose, pullulan, carrageenan, scleroglucan, chitin, curdulan, levan, amylopectin, starch, resistant starch, azo compounds being degraded by azo bonds splitting bacteria, and combinations thereof. In a preferred embodiment of the present invention, the delayed release coating comprises a combination of poly(methacrylic acid, methyl methacrylate) 1:2 (Eudragit® S) and resistant starch. The delayed release coating comprising at least one pH sensitive (enteric) polymer, e.g. poly(methacrylic acid, methyl methacrylate) 1:2, and at least one polysaccharide, e.g. resistant starch, may be dispersed in an organic solvent, a mixture of organic solvents or a mixture of at least one organic solvent and water, and then applied to the multiparticulate solid dosage form e.g. by spray coating, preferably fluidized-bed spray coating.

In another embodiment, the delayed release coating comprises i) an inner coating comprising a partially neutralized pH sensitive (enteric) polymer adjusted to pH 8 (e.g. neutralized poly(methacrylic acid, methyl methacrylate) 1:2 adjusted to pH 8) and containing a buffer salt, and ii) an outer coating comprising a combination of at least one pH sensitive (enteric) polymer, e.g. poly(methacrylic acid, methyl methacrylate) 1:2 (e.g. Eudragit® S), and at least one polysaccharide selected from chondroitin sulfate, cyclodextrin, chitosan, dextran, arabinogalactan, amylose, pullulan, carrageenan, scleroglucan, chitin, curdulan, levan, amylopectin, starch, resistant starch, azo compounds being degraded by azo bonds splitting bacteria, and combinations thereof. In another preferred embodiment, the delayed release coating comprises an inner coating comprising a partially neutralized pH sensitive (enteric) polymer adjusted to pH 8 (e.g. neutralized poly(methacrylic acid, methyl methacrylate) 1:2 adjusted to pH 8) and containing a buffer salt, and an outer coating comprising a combination of poly(methacrylic acid, methyl methacrylate) 1:2 (e.g. Eudragit® S) and resistant starch. Other preferred embodiment for the delayed release coating can be found among the embodiments disclosed in WO2007122374A2.

The inventive method in one of the embodiments described above ensures that the activity and stability of the at least one antibody or functional fragment thereof is preserved in the particles. The stability and activity of an antibody or fragment thereof can be estimated for example by determining the fraction of an antibody or functional fragment thereof present as dimers and other aggregates. According to one embodiment of the present invention, the fraction of total content of antibody or functional fragment thereof present in the particles or in the multiparticulate solid dosage form as dimers and other aggregates does not exceed more than 20 %, preferably 15%, more preferably 12 %, even more preferably 10 %, even more preferably 8%, even more preferably 7 %, even more preferably 8%, even more preferably 5 %, 3 %, 2 % or 1.5 %, the fraction of total antibody or functional fragment thereof present as dimers and other aggregates at the time of adding the antibody or functional fragment thereof to the feed liquid. Methods to determine the fraction of a polypeptide present as dimers and other aggregates are known in the art, and include for example Size Exclusion Chromatography (SEC).

The stability and activity of an antibody or functional fragment thereof can also be estimated for example by determining the fraction of an antibody or functional fragment thereof present as fragments of the full-length antibody or functional fragment thereof. Therefore, in another embodiment of the present invention, the fraction of total content of antibody or functional fragment thereof present in the particles or in the multiparticulate solid dosage form as fragments of the full-length antibody or functional fragment thereof does not increase substantially compared to the time of adding the antibody or functional fragment thereof to the feed liquid. The term "substantially" as used herein refers to a deviation from a stated condition by not more than 50%, preferably not more than 20 %, more preferably not more than 15 %, even more preferably not more than 10 %, even more preferably not more than 7%, even more preferably not more than 5 %, 3 %, 2 %, 1.5 %, or 1 %.

In a further embodiment of the present invention, the fraction of total content of antibody or functional fragment thereof present in the particles or in the multiparticulate solid dosage form as fragments of the full-length antibody or functional fragment thereof does not exceed by more than 20 %, preferably 15%, more preferably 12 %, even more preferably 10 %, even more preferably 8%, even more preferably 6 %, 5 %, 3 %, 2 %, or 1.5 %, the fraction of total content of antibody or functional fragment thereof present as fragments of the full-length antibody or functional fragment thereof at the time of adding the antibody or functional fragment thereof to the feed liquid. Methods to determine the fraction of an antibody or functional fragment thereof present as fragments of the full-length antibody or functional fragment thereof are known in the art, and include for example microchip electrophoresis analysis.

In yet another further embodiment of the present invention the multiparticulate solid dosage forms prepared by the inventive method are for use in the treatment of a disease in the gastrointestinal tract (gastrointestinal diseases), preferably in the ileum and the large intestine. Such diseases include e.g. IBD, cancer (such as colorectal cancer or small intestine cancer), infections (such as Clostridium difficile infection) of the small intestine and the colon, and diarrhea. In a preferred embodiment of the present invention the particles prepared by the inventive method are for use in the treatment of IBD, e.g. Crohn's disease or ulcerative colitis.

In addition to a method for preparing particles and multiparticulate solid dosage forms as described in the embodiments above, the present invention further relates to particles obtainable by the method of the present invention as defined by any one of the embodiments described above. The present invention further relates to a multiparticulate solid dosage form obtainable by the method of the present invention as defined by any one of the embodiments described above. The inventive multiparticulate solid dosage forms may be in the form of pellets, beads, spheres, mini spheres, spheroids granules, beads, tablets, mini tablets, sachets, stick packs, straw devices (Xstraw®), capsules and the like.

Furthermore, the present invention relates to said particles or multiparticulate solid dosage forms for use in the treatment of a gastrointestinal diseases, preferably an IBD, colorectal cancer, small intestine cancer, celiac disease or gastrointestinal infections (e.g. Clostridium difficile infection), more preferably an IBD, e.g. Crohn's disease or ulcerative colitis. The present invention also relates to said particles, solid dosage forms or multiparticulate solid dosage forms prepared by the inventive method described above for use in the topical treatment in the gastrointestinal tract of a patient. Finally the present invention relates to said inventive said particles or multiparticulate solid dosage forms for use in the treatment of a patient suffering from a gastrointestinal disease, preferably an IBD, colorectal cancer, small intestine cancer, celiac disease or gastrointestinal infections, more preferably IBD.

According to a further aspect the present invention relates to a particle, comprising at least one antibody or functional fragment thereof as active agent, a buffer and at least one sustained release polymer, and optionally a plasticizer and/or a surfactant and/or filler, obtainable by spray drying .In one embodiment the particle is obtainable by spray drying comprising the steps of a) preparing a feed liquid comprising the at least one antibody or functional fragment thereof, the buffer and the at least one sustained release polymer, and optionally a plasticizer and/or a surfactant and/or filler, all in an aqueous suspension; b) spraying the feed liquid through a nozzle into a primary drying chamber to form droplets, wherein the droplets are dispersed in a drying gas; c) drying the droplets, by evaporating at least a portion of the liquid in the droplets into the drying gas, to form particles dispersed in the drying gas; and d) directing the drying gas with the particles dispersed therein, through a particle concentration means to recover the particles.

According to yet another aspect, the present invention relates to a multiparticulate solid dosage form, comprising a plurality of particles, each of the particles obtainable by the inventive method as described in any of the embodiments above, wherein the multiparticulate solid dosage form preferably is a sachet, stick pack, tablet, mini tablet, pellet, bead, sphere, spheroid, mini sphere, straw device (X-straw®), or capsule.

According to yet another aspect the present invention relates to a multiparticulate solid dosage form, comprising a plurality of particles, wherein each particle comprises at least one antibody or functional fragment thereof as active agent, a buffer and at least one sustained release polymer, and optionally a plasticizer and/or a surfactant and/or filler, and is obtainable by spray drying. In one embodiment the particles of the multiparticulate solid dosage form are obtainable by spray drying comprising the steps of a) preparing a feed liquid comprising the at least one antibody or functional fragment thereof, the buffer and the at least one sustained release polymer, and optionally a plasticizer and/or a surfactant and/or filler, all in an aqueous suspension; b) spraying the feed liquid through a nozzle into a primary drying chamber to form droplets, wherein the droplets are dispersed in a drying gas; c) drying the droplets, by evaporating at least a portion of the liquid in the droplets into the drying gas, to form particles dispersed in the drying gas; and d)directing the drying gas with the particles dispersed therein, through a particle concentration means to recover the particles.

According another embodiment the inventive particle or the inventive multiparticulate solid dosage is suitable for use in the topical treatment of a gastrointestinal disease, preferably IBD, celiac disease, a gastrointestinal infection, a colorectal cancer or a small intestine cancer, more preferably an IBD.

According to yet another embodiment, the at least one antibody or functional fragment thereof comprised in the inventive particle or comprised in the particles comprised in the inventive multiparticulate solid dosage form is as defined in any one of the embodiments regarding the inventive method for the preparation of the particles described above. According to yet another embodiment, the buffer comprised in the inventive particle or comprised in the particles comprised in the inventive multiparticulate solid dosage form is as defined in any one of the embodiments regarding the inventive method for the preparation of the particles described above. According to yet another embodiment, the at least one sustained release polymer comprised in the inventive particle or comprised in the particles comprised in the inventive multiparticulate solid dosage form is as defined in any one of the embodiments regarding the inventive method for the preparation of the particles described above. According to yet another embodiment, the plasticizer and/or surfactant and/or filler optionally comprised in the inventive particle or comprised in the particles comprised in the inventive multiparticulate solid dosage form is/are as defined in any one of the embodiments regarding the inventive method for the preparation of the particles described above.

According to a further embodiment, the inventive particle or the particles comprised in the inventive multiparticulate solid dosage form has/have the properties as defined in any of the embodiment described above with regard to the inventive method for the preparation of the particles.

According to another embodiment the individual inventive particles or individual particles of the multiparticulate solid dosage form allow the recovery of at least 80 %, preferably at least 85%, more preferably at least 93 %, even more preferably at least 95 %, even more preferably at least 97%, even more preferably at least 98%, of the at least one antibody or functional fragment thereof from the particles. According to yet another embodiment the individual inventive particles or individual particles of the multiparticulate solid dosage form allow the recovery of at least 80 %, preferably at least 85%, more preferably at least 93 %, even more preferably at least 95 %, even more preferably at least 97%, even more preferably at least 98%, of the at least one antibody or functional fragment thereof from the particles, within 4 h, or 6 h, or 8 h, or 10 h, or 12 h, or 14 h, or 16 h, or 18 h, or 20 h, or 22 h, or 24 h, or 26 h, or 28 h, or 30 h, or 32 h, or 34 h, or 36 h, etc., of continuously immersing the particles or the multiparticulate solid dosage form in an aqueous solution under continuous agitation (sustained release).

According to one embodiment, at least 80%, preferably 90%, more preferably 95%, by number of those particles comprised in the inventive multiparticulate solid dosage form, have a median aspect ratio between 0.6 and 1.8, the aspect ratio being defined as particle length along the predetermined axis divided by the smallest cross-sectional dimension. According to another embodiment the median aspect ratio is above 0.7 preferably above 0.8, more preferably above 0.9, and below 1.7, preferably 1.6, more preferably below 1.5, more preferably 1.4, even more preferably below 1.3, even more preferably below 1.2, most preferably about 1. According to yet another embodiment the particles comprised in the inventive multiparticulate solid dosage form have a span of aspect ratio of less than 0.9, preferably less than 0.8, more preferably less than 0.7, even more preferably less than 0.6, most preferably less than 0.5. For further details regarding aspect ratio, predetermined axis, predetermined cross-sectional profile and span (including definitions and embodiments), it is referred to disclosure of EP 2 512 453.

According to a further embodiment the inventive multiparticulate solid dosage form is prepared by compression, encapsulation or extrusion-spheronization including spray-dried particles. According to yet a further embodiment the multiparticulate solid dosage form comprises a delayed release coating, which is applied as a further coating.

### EXAMPLES

### Materials and methods applied in the examples

*Citrate-TRIS buffer pH 7 preparation:* A solution of sodium citrate 100 mM (2.942 g and completed to 100.0 mL with purified water) was prepared. A solution of citric acid 100 mM (3.842 g dissolved and diluted to 200.0 ml with purified water) was prepared. The pH of the citric acid solution was adjusted to 3.5 with the sodium citrate solution. A TRIS solution 1 M (12.114 g and completed to 100.0 ml with purified water) was prepared. The pH of the citrate buffer was adjusted to pH 7.0 with the TRIS solution.

**Table 1: Preparation of spray-dried adalimumab particles**

| *Example* | *Polymer* | *Polymer:Antibody ratio (w*/*w)* | *Antibody loading (%)* | *Total solid (%)* | *Inlet T (°C)* | *Nozzle air (l*/*min)* |
|---|---|---|---|---|---|---|
| *Comparative Example 1* | *n.a.* | *n.a.* | *100* | *5.9* | *100* | *6.0* |
| *Comparative Example 2* | *n.a.* | *n.a.* | *100* | *5.9* | *100* | *4.1* |
| *Comparative Example 3* | *n.a.* | *n.a.* | *100* | *5.9* | *120* | *4.1* |
| *Example 1* | *Eudragit*® *RS 30D* | *21.9* | *3.9* | *18.9* | *100* | *6.5* |
| *Example 2* | *Eudragit*® *RS 30D* | *21.9* | *3.9* | *18.9* | *120* | *4.1* |
| *Example 3* | *Eudragit*® *RS 30D* | *21.9* | *3.9* | *9.3* | *100* | *6.5* |
| *Example 4* | *Kollicoat*® *SR 30D* | *89.7* | *1.1* | *29.4* | *120* | *6.5* |

### Dissolution of adalimumab spray-dried particles

A quantity of adalimumab loaded particles was placed in a 5 ml cryo tube and 2.0 ml of buffer was added to yield a theoretical 1 mg/ml adalimumab concentration, based on the theoretical calculated adalimumab loading. Citrate-TRIS buffer pH 7 was used unless stated otherwise. Samples are agitated during the entire duration of the experiment. Supernatant samples (100 µl) were taken at predetermined time points, centrifuged and the supernatant was analysed in terms of total protein content or by SEC, presence of aggregates (SEC) and fragmentation (electrophoresis) whenever specified.

*Microchip Electrophoresis analysis (fragments):* The supernatant (2 µl) of samples containing adalimumab were tested for the presence of fragments by microchip gel electrophoresis under non-reducing conditions. In all experiments a positive control of adalimumab 1 mg/ml in citrate-TRIS buffer pH 7 was used.

*Size exclusion chromatography (aggregation):* The supernatant of samples containing adalimumab was tested for the presence of aggregates (dimers, oligomers) by size exclusion chromatography (SEC). In all experiments a positive control of adalimumab 1 mg/ml in citrate-TRIS buffer pH 7 was used.

*Ionic exchange chromatography (surface charge):* the supernatant of samples containing adalimumab was tested for the presence of basic and acidic (deaminated) variants by ion exchange chromatography (IEX). A strong cation exchange column with a salt gradient was utilized. In all experiments a positive control of adalimumab 1 mg/mL in citrate-TRIS buffer pH 7 was used.

### Results

### Experiment 1 - Characteristics of a spray-dried model antibody

The suitability of spray-drying and the effect of some process parameters to obtain a solid form of adalimumab were investigated. Briefly, inlet temperature and nozzle air were varied in the ranges 100-120 °C and 4.1-6.0 l/min, respectively. All the combinations of the listed process parameters resulted in free-flowing and spherical particles. Physico-chemical properties of the spray-dried antibody were then assessed in terms of aggregation, fragmentation and surface charge (Figure 1A-C). No significant increase in dimer content (Figure 1A) in comparison to the positive control (1.0 mg/mL standard - liquid antibody) was observed for the dried samples. Similarly, neither significant increase of fragments (Figure 1 B) nor modification of surface charge (Figure 1C) was measured in comparison to the reference sample.

### Experiment 2 - Co-spray-drying of adalimumab and Eudragit RS 30D: Compatibility study

The suitability of spray-drying and the effect of some process parameters to obtain co-processed particles of adalimumab and Eudragit® RS 30D were investigated.
Figure 2 shows the relative dimer to monomer content (determined by HPLC-SEC) and the relative fragmentation profile (determined by microchip electrophoresis) of adalimumab released in citrate-TRIS buffer pH 7 after 24 hours from particles obtained by spray-drying, comprising adalimumab and Eudragit® RS 30D (polymer:adalimumab mass ratio of 21.9) using a feed solution containing 18.8 wt.-% total solids. The feed suspension was processed with an inlet air temperature of 100 °C (Example 1) or 120 °C (Example 2). The nozzle air was varied in the ranges of 4.1-6.5 l/min, respectively. All the combination of the listed process parameters resulted in free-flowing and spherical particles. Physico-chemical properties of the spray-dried antibody were then assessed in terms of aggregation and fragmentation (Figure 2A-B). No significant increase in dimer content (Figure 2A) in comparison to the positive control (1.0 mg/mL standard - liquid antibody) was observed for the dried samples. Similarly, no significant increase of fragments (Figure 2B) was measured in comparison to the reference sample.

### Experiment 3 - Co-spray-drying of adalimumab and Eudragit RS 30D: Preparation of particles able to sustain the release of adalimumab

Figure 3 shows the adalimumab release kinetics from particles obtained by co-spray-drying, comprising adalimumab and Eudragit® RS 30D (polymer:adalimumab mass ratio of 21.9) using a feed solution containing 9.3 wt.-% total solids, after reconstitution in citrate-TRIS pH 7 (Example 3). Average and standard deviation of 3 measurements are shown. Adalimumab release from co-processed particles can be controlled over time by Eudragit® RS 30D in the composition here described.

### Experiment 4 - Co-spray-drying of adalimumab and Kollicoat SR 30D: Compatibility study

The suitability of spray-drying to obtain co-processed particles of adalimumab and Kollicoat® SR 30D were investigated. Figure 4 shows the relative dimer to monomer content (determined by HPLC-SEC) and the relative fragmentation profile (determined by microchip electrophoresis) of adalimumab released in citrate-TRIS buffer pH 7 after 24 hours from particles obtained by spray-drying, comprising adalimumab and Kollicoat SR 30D (polymer:adalimumab mass ratio of 89.7) using a feed solution containing 29.4 % wt total solids (Example 4).. The feed suspension was processed with an inlet air temperature of 120°C with a nozzle air of 6.5 l/min. The resultant spray-dried particles were free-flowing and spherical in shape. Physico-chemical properties of the spray-dried antibody were then assessed in terms of aggregation and fragmentation (Figure 4A-B). No significant increase in dimer content (Figure 4A) in comparison to the positive control (1.0 mg/mL standard - liquid antibody) was observed for the dried samples. Similarly, no significant increase of fragments (Figure 4B) was measured in comparison to the reference sample.

## Claims

1. A method for preparing particles comprising at least one antibody or functional fragment thereof as active agent, a buffer and at least one sustained release polymer, by spray drying; the method comprising the steps of
a) preparing a feed liquid comprising the at least one antibody or functional fragment thereof, the buffer and the at least one sustained release polymer, all in an aqueous suspension;
b) spraying the feed liquid through a nozzle into a primary drying chamber to form droplets, wherein the droplets are dispersed in a drying gas;
c) drying the droplets, by evaporating at least a portion of the liquid in the droplets into the drying gas, to form particles dispersed in the drying gas; and
d) directing the drying gas with the particles dispersed therein, through a particle concentration means to recover the particles.

2. Method according to claim 1, wherein the at least one sustained release polymer is selected from the group consisting of poly(ethyl acrylate, methyl methacrylate, trimethylammonioethyl methacrylate chloride) 1 : 2 : 0.1 (Eudragit® RS 30D); poly(ethyl acrylate, methyl methacrylate, trimethylammonioethyl methacrylate chloride) 1 : 2 : 0.2 (e.g. Eudragit® RL 30D); poly(ethylacrylate, methylmethacrylate) 2:1 (e.g. Eudragit® NE 30D, Eudragit® NE 40D, Eudragit® NM 30D); ethylcellulose aqueous dispersions (such as Aquacoat® ECD and Surelease®); polyvinyl acetate; polyvinyl acetate aqueous dispersion stabilized with povidone and sodium lauryl sulfate; aqueous dispersion consisting of about 27% polyvinyl acetate, 2.7% povidone and 0.3% sodium lauryl sulfate (Kollicoat® SR 30D); hydroxypropyl methylcellulose (HPMC); HPMC 2208 type with a viscosity at 2 wt.-% in water at 20°C between 3 and 1,000 mPa·s, preferably HPMC 2208 type with a viscosity at 2 wt.-% in water at 20°C between 78 and 117 mPa·s (. Methocel® K100); and combinations thereof.

3. Method according to any of the preceding claims, wherein the feed liquid comprises
- 5 to 95 wt.-%, preferably 75 to 90 wt.-%, more preferably about 83 to 85 wt.-%, of the at least one sustained release polymer; and/or
- 0.01 to 30 wt.-%, preferably 0.1 to 15 wt.-%, more preferably about 3.5 to 4.5 wt.-%, of the at least one antibody or functional fragment thereof;
relative to the total solid content of the feed liquid.

4. Method according to any of the preceding claims, wherein the feed liquid comprises a mass ratio of the at least one sustained release polymer to the at least one antibody or functional fragment thereof of between 0.5 and 100, preferably 5 and 90, more preferably 15 and 85, even more preferably 20 and 50, even more preferably 21 and 30.

5. Method according to any of the preceding claims, wherein the feed liquid comprises at least one filler, which is selected from dextrose, lactose, lactose monohydrate, lactose anhydrous, mannitol, sorbitol, xylitol, sucrose, glucose, raffinose, trehalose, dicalcium phosphate, oxides of magnesium or silicon, titanium carbonate, calcium carbonate, magnesium phosphate, porous calcium phosphate or porous magnesium phosphate, amino acids such as arginine, histidine, glycine, alanine, lysine, proline, leucine, glutamic acid, serine, aspartic acid and asparagine, and respective salts thereof, propylene glycol, polyethylene glycol, and combinations thereof; preferably dextrose, sucrose, mannitol, sorbitol, xylitol, trehalose, amino acids such as arginine, histidine, glycine, alanine, lysine, proline, leucine, glutamic acid, serine, aspartic acid and asparagine, and respective salts thereof, dicalcium phosphate, and combinations thereof.

6. Method according to any of the preceding claims, wherein the feed liquid comprises at least one further excipient selected from plasticizers, surfactants, pore forming agents, viscosity-reducing agents, divalent cations, antioxidants, humectants, protective colloids, dyes, and combinations thereof.

7. Method according to any of the preceding claims, wherein during step b)
- the inlet temperature of the drying gas is between 50 °C and 200 °C, preferably between 80°C and 190°C, more preferably between 90 °C and 170 °C, even more preferably between 90 °C and 150 °C; and/or
- the outlet temperature of the drying gas with the particles dispersed therein is between 25 °C and 120 °C, preferably between 40°C and 100°C, more preferably between 45 °C and 90 °C, even more preferably between 45 °C and 80 °C.

8. Method according to any of the preceding claims, wherein the particles after step d) have a particle-size distribution (PSD) D50 of 0.5-200 µm, preferably 1-100 µm, more preferably 5-75 µm, even more preferably 5-50 µm.

9. Method according to any of the preceding claims, wherein the at least one antibody or functional fragment thereof is selected from antibodies specific to tumor necrosis factor alpha (TNFα) and functional fragments thereof; antibodies specific to α4β7 integrin and functional fragments thereof, antibodies specific to CD3, CD4 or CD20 and functional fragments thereof; antibodies specific to interleukin 6 (IL-6), interleukin 12 (IL-12), interleukin 13 (IL-13), interleukin 23 (IL-23), or to their receptors, and functional fragments thereof; antibodies specific to CXCL10/IP-10 and functional fragments thereof; and antibodies specific to p40 protein subunit and functional fragments thereof.

10. Method according to any of the preceding claims, comprising the further step of preparing a multiparticulate solid dosage form, preferably in the form of a tablet, mini tablet, pellet, bead, sphere, spheroid, mini sphere, or capsule, comprising a plurality of particles.

11. Method according to claim 10, further comprising the step of applying at least one additional coating in the form of a delayed release coating, and wherein the multiparticulate solid dosage form is for oral administration.

12. Method according to claim 11, wherein the delayed release coating comprises at least one component selected from the group consisting of poly vinyl acetate phthalate, cellulose acetate trimellitate, hydroxypropyl methylcellulose phthalate HP-50, HP-55 or HP-55S, cellulose acetate phthalate, hydroxypropyl methylcellulose acetate succinate (HPMCAS), poly(methacrylic acid, ethyl acrylate) 1:1, poly(methacrylic acid, methyl methacrylate) 1:1, poly(methacrylic acid, methyl methacrylate) 1:2, chondroitin sulfate, pectin, guar gum, chitosan, inulin, lactulose, raffinose, stachyose, alginate, dextran, xanthan gum, locust bean gum, arabinogalactan, amylose, cyclodextrin, pullulan, carrageenan, scleroglucan, chitin, curdulan, levan, amylopectin, starch, resistant starch, azo compounds being degraded by azo bonds splitting bacteria, and combinations thereof.

13. Method according to claim 11 or 12, wherein, upon oral administration of the multiparticulate solid dosage form, the release of the antibody or functional fragment thereof starts in the terminal ileum, the ileocolonic region, the ascending colon, transverse colon or the descending colon.

14. A particle or multiparticulate solid dosage obtainable by the method of any of claims 1 to 13.

15. Particle or multiparticulate solid dosage form according to claim 14 for use in the treatment of a patient suffering from a gastrointestinal disease.
